# EUROPEAN PATENT SPECIFICATION

(11) **EP 2 417 457 B1**
(45) Date of publication and mention of the grant of the patent: **13.05.2015**
(21) Application number: 10712791.2
(22) Date of filing: 31.03.2010
(51) Int. Cl.: G01N 33/574, C07K 14/00, A61K 49/00

(54) **PROTEIN-BASED METHODS AND COMPOSITIONS FOR THE DIAGNOSIS OF COLORECTAL ADENOCARCINOMA**
VERFAHREN UND ZUSAMMENSETZUNGEN AUF PROTEINBASIS ZUR DIAGNOSE DES KOLEKTORALEN ADENOKARZINOMS
Procédés à base de protéine et compositions pour le diagnostic d'adénocarcinome colorectal

(30) Priority: 07.04.2009 EP 09157464
(43) Date of publication of application: 15.02.2012
(73) Proprietor: Vereniging voor Christelijk Hoger Onderwijs, Wetenschappelijk Onderzoek en Patiëntenzorg, 1081 HV Amsterdam (NL)
(72) Inventor: DE WIT, Meike, NL-5656 AE Eindhoven (NL); FIJNEMAN, Remondus, J., A., NL-5656 AE Eindhoven (NL); JIMENEZ, Cornelia, R., NL-5656 AE Eindhoven (NL); MEIJER, Gerrit, A., NL-5656 AE Eindhoven (NL)
(74) Representative: Kroeze, Johannes Antonius
(86) International application number: PCT/IB2010/051395
(87) International publication number: WO 2010/119362

(56) References cited:
- WO-A-02/057450
- CARRER A ET AL: "Expression profiling of angiogenic genes for the characterisation of colorectal carcinoma" EUROPEAN JOURNAL OF CANCER, PERGAMON PRESS, OXFORD, GB, vol. 44, no. 12, 1 August 2008 (2008-08-01), pages 1761-1769, XP023521010 ISSN: 0959-8049 [retrieved on 2008-07-23]
- YANAMOTO SOUICHI ET AL: "Clinicopathologic significance of EpCAM expression in squamous cell carcinoma of the tongue and its possibility as a potential target for tongue cancer gene therapy." ORAL ONCOLOGY OCT 2007, vol. 43, no. 9, October 2007 (2007-10), pages 869-877, XP022235347 ISSN: 1368-8375
- CURTET C ET AL: "Gd-25 DTPA-MAb, a potential NMR contrast agent for MRI in the xenografted nude mouse: preliminary studies." INTERNATIONAL JOURNAL OF CANCER. SUPPLEMENT = JOURNAL INTERNATIONAL DU CANCER. SUPPLEMENT 1988, vol. 2, 1988, pages 126-132, XP008111248 ISSN: 0898-6924
- CHANG H ET AL: "Using a combination of cytochrome P450 1B1 and beta-catenin for early diagnosis and prevention of colorectal cancer" CANCER DETECTION AND PREVENTION, ELSEVIER SCIENCE, vol. 29, no. 6, 1 January 2005 (2005-01-01), pages 562-569, XP025263594 ISSN: 0361-090X [retrieved on 2005-01-01]
- THIEROLF MICHAEL ET AL: "Towards a comprehensive proteome of normal and malignant human colon tissue by 2-D-LC-ESI-MS and 2-DE proteomics and identification of S100A12 as potential cancer biomarker" PROTEOMICS CLINICAL APPLICATIONS, vol. 2, no. 1, January 2008 (2008-01), pages 11-22, XP002543998 ISSN: 1862-8346
- HALLAK RANA ET AL: "p53 genetic alterations, protein expression and autoantibodies in human colorectal carcinoma. A comparative study" INTERNATIONAL JOURNAL OF ONCOLOGY, vol. 12, no. 4, April 1998 (1998-04), pages 785-791, XP008111315 ISSN: 1019-6439
- SHIMIZU M ET AL: "TUMOR IMAGING WITH ANTI-CEA ANTIBODY LABELED 19F EMULSION", MAGNETIC RESONANCE IN MEDICINE, ACADEMIC PRESS, DULUTH, MN, US, vol. 5, 1 January 1987 (1987-01-01), pages 290-295, XP001033873, ISSN: 0740-3194
- REIMER P ET AL: "RECEPTOR IMAGING: APPLICATION TO MR IMAGING OF LIVER CANCER", RADIOLOGY, RADIOLOGICAL SOCIETY OF NORTH AMERICA, OAK BROOK,IL, US, vol. 177, no. 3, 1 December 1990 (1990-12-01), pages 729-734, XP000606058, ISSN: 0033-8419

### SEQUENCE LISTING

<110> Vereniging voor Christelijk Hoger Onderwijs, Wetenschappelijk Onderzoek en Patientenzorg
<120> Protein-based methods and compositions for the diagnosis of colorectal adenocarcinoma
<130> PH012911WO1
<150> EP 09157464.0
   <151> 2009-04-07
<160> 32
<170> PatentIn version 3.5
<210> 1
   <211> 1896
   <212> PRT
   <213> Homo sapiens
<400> 1
<210> 2
   <211> 541
   <212> PRT
   <213> Homo sapiens
<400> 2
   Met Val Ala Asp Pro Pro Arg Asp Ser Lys Gly Leu Ala Ala Ala Glu
   1               5                   10                  15
   Pro Thr Ala Asn Gly Gly Leu Ala Leu Ala Ser Ile Glu Asp Gln Gly
               20                  25                  30
   Ala Ala Ala Gly Gly Tyr Cys Gly Ser Arg Asp Gln Val Arg Arg Cys
           35                  40                  45
   Leu Arg Ala Asn Leu Leu Val Leu Leu Thr Val Val Ala Val Val Ala
       50                  55                  60
   Gly Val Ala Leu Gly Leu Gly Val Ser Gly Ala Gly Gly Ala Leu Ala
   65                  70                  75                  80
   Leu Gly Pro Glu Arg Leu Ser Ala Phe Val Phe Pro Gly Glu Leu Leu
                   85                  90                  95
   Leu Arg Leu Leu Arg Met Ile Ile Leu Pro Leu Val Val Cys Ser Leu
               100                 105                 110
   Ile Gly Gly Ala Ala Ser Leu Asp Pro Gly Ala Leu Gly Arg Leu Gly
           115                 120                 125
   Ala Trp Ala Leu Leu Phe Phe Leu Val Thr Thr Leu Leu Ala Ser Ala
       130                 135                 140
   Leu Gly Val Gly Leu Ala Leu Ala Leu Gln Pro Gly Ala Ala Ser Ala
   145                 150                 155                 160
   Ala Ile Asn Ala Ser Val Gly Ala Ala Gly Ser Ala Glu Asn Ala Pro
                   165                 170                 175
   Ser Lys Glu Val Leu Asp Ser Phe Leu Asp Leu Ala Arg Asn Ile Phe
               180                 185                 190
   Pro Ser Asn Leu Val Ser Ala Ala Phe Arg Ser Tyr Ser Thr Thr Tyr
           195                 200                 205
   Glu Glu Arg Asn Ile Thr Gly Thr Arg Val Lys Val Pro Val Gly Gln
       210                 215                 220
   Glu Val Glu Gly Met Asn Ile Leu Gly Leu Val Val Phe Ala Ile Val
   225                 230                 235                 240
   Phe Gly Val Ala Leu Arg Lys Leu Gly Pro Glu Gly Glu Leu Leu Ile
                   245                 250                 255
   Arg Phe Phe Asn Ser Phe Asn Glu Ala Thr Met Val Leu Val Ser Trp
               260                 265                 270
   Ile Met Trp Tyr Ala Pro Val Gly Ile Met Phe Leu Val Ala Gly Lys
           275                 280                 285
   Ile Val Glu Met Glu Asp Val Gly Leu Leu Phe Ala Arg Leu Gly Lys
       290                 295                 300
   Tyr Ile Leu Cys Cys Leu Leu Gly His Ala Ile His Gly Leu Leu Val
   305                 310                 315                 320
   Leu Pro Leu Ile Tyr Phe Leu Phe Thr Arg Lys Asn Pro Tyr Arg Phe
                   325                 330                 335
   Leu Trp Gly Ile Val Thr Pro Leu Ala Thr Ala Phe Gly Thr Ser Ser
               340                 345                 350
   Ser Ser Ala Thr Leu Pro Leu Met Met Lys Cys Val Glu Glu Asn Asn
           355                 360                 365
   Gly Val Ala Lys His Ile Ser Arg Phe Ile Leu Pro Ile Gly Ala Thr
       370                 375                 380
   Val Asn Met Asp Gly Ala Ala Leu Phe Gln Cys Val Ala Ala Val Phe
   385                 390                 395                 400
   Ile Ala Gln Leu Ser Gln Gln Ser Leu Asp Phe Val Lys Ile Ile Thr
                   405                 410                 415
   Ile Leu Val Thr Ala Thr Ala Ser Ser Val Gly Ala Ala Gly Ile Pro
               420                 425                 430
   Ala Gly Gly Val Leu Thr Leu Ala Ile Ile Leu Glu Ala Val Asn Leu
           435                 440                 445
   Pro Val Asp His Ile Ser Leu Ile Leu Ala Val Asp Trp Leu Val Asp
       450                 455                 460
   Arg Ser Cys Thr Val Leu Asn Val Glu Gly Asp Ala Leu Gly Ala Gly
   465                 470                 475                 480
   Leu Leu Gln Asn Tyr Val Asp Arg Thr Glu Ser Arg Ser Thr Glu Pro
                   485                 490                 495
   Glu Leu Ile Gln Val Lys Ser Glu Leu Pro Leu Asp Pro Leu Pro Val
               500                 505                 510
   Pro Thr Glu Glu Gly Asn Pro Leu Leu Lys His Tyr Arg Gly Pro Ala
   515 520 525
   Gly Asp Ala Thr Val Ala Ser Glu Lys Glu Ser Val Met
   530 535 540
<210> 3
   <211> 509
   <212> PRT
   <213> Homo sapiens
<400> 3
   Met Gly Cys Ser Ala Lys Ala Arg Trp Ala Ala Gly Ala Leu Gly Val
   1               5                   10                  15
   Ala Gly Leu Leu Cys Ala Val Leu Gly Ala Val Met Ile Val Met Val
               20                  25                  30
   Pro Ser Leu Ile Lys Gln Gln Val Leu Lys Asn Val Arg Ile Asp Pro
           35                  40                  45
   Ser Ser Leu Ser Phe Asn Met Trp Lys Glu Ile Pro Ile Pro Phe Tyr
       50                  55                  60
   Leu Ser Val Tyr Phe Phe Asp Val Met Asn Pro Ser Glu Ile Leu Lys
   65                  70                  75                  80
   Gly Glu Lys Pro Gln Val Arg Glu Arg Gly Pro Tyr Val Tyr Arg Glu
                   85                  90                  95
   Phe Arg His Lys Ser Asn Ile Thr Phe Asn Asn Asn Asp Thr Val Ser
               100                 105                 110
   Phe Leu Glu Tyr Arg Thr Phe Gln Phe Gln Pro Ser Lys Ser His Gly
           115                 120                 125
   Ser Glu Ser Asp Tyr Ile Val Met Pro Asn Ile Leu Val Leu Gly Ala
       130                 135                 140
   Ala Val Met Met Glu Asn Lys Pro Met Thr Leu Lys Leu Ile Met Thr
   145                 150                 155                 160
   Leu Ala Phe Thr Thr Leu Gly Glu Arg Ala Phe Met Asn Arg Thr Val
                   165                 170                 175
   Gly Glu Ile Met Trp Gly Tyr Lys Asp Pro Leu Val Asn Leu Ile Asn
               180                 185                 190
   Lys Tyr Phe Pro Gly Met Phe Pro Phe Lys Asp Lys Phe Gly Leu Phe
   195 200 205
   Ala Glu Leu Asn Asn Ser Asp Ser Gly Leu Phe Thr Val Phe Thr Gly
   210 215 220
   Val Gln Asn Ile Ser Arg Ile His Leu Val Asp Lys Trp Asn Gly Leu
   225 230 235 240
   Ser Lys Val Asp Phe Trp His Ser Asp Gln Cys Asn Met Ile Asn Gly
   245 250 255
   Thr Ser Gly Gln Met Trp Pro Pro Phe Met Thr Pro Glu Ser Ser Leu
   260 265 270
   Glu Phe Tyr Ser Pro Glu Ala Cys Arg Ser Met Lys Leu Met Tyr Lys
   275 280 285
   Glu Ser Gly Val Phe Glu Gly Ile Pro Thr Tyr Arg Phe Val Ala Pro
   290 295 300
   Lys Thr Leu Phe Ala Asn Gly Ser Ile Tyr Pro Pro Asn Glu Gly Phe
   305 310 315 320
   Cys Pro Cys Leu Glu Ser Gly Ile Gln Asn Val Ser Thr Cys Arg Phe
   325 330 335
   Ser Ala Pro Leu Phe Leu Ser His Pro His Phe Leu Asn Ala Asp Pro
   340 345 350
   Val Leu Ala Glu Ala Val Thr Gly Leu His Pro Asn Gln Glu Ala His
   355 360 365
   Ser Leu Phe Leu Asp Ile His Pro Val Thr Gly Ile Pro Met Asn Cys
   370 375 380
   Ser Val Lys Leu Gln Leu Ser Leu Tyr Met Lys Ser Val Ala Gly Ile
   385 390 395 400
   Gly Gln Thr Gly Lys Ile Glu Pro Val Val Leu Pro Leu Leu Trp Phe
   405 410 415
   Ala Glu Ser Gly Ala Met Glu Gly Glu Thr Leu His Thr Phe Tyr Thr
   420 425 430
   Gln Leu Val Leu Met Pro Lys Val Met His Tyr Ala Gln Tyr Val Leu
   435 440 445
   Leu Ala Leu Gly Cys Val Leu Leu Leu Val Pro Val Ile Cys Gln Ile
   450 455 460
   Arg Ser Gln Glu Lys Cys Tyr Leu Phe Trp Ser Ser Ser Lys Lys Gly
   465                 470                 475                 480
   Ser Lys Asp Lys Glu Ala Ile Gln Ala Tyr Ser Glu Ser Leu Met Thr
                   485                 490                 495
   Ser Ala Pro Lys Gly Ser Val Leu Gln Glu Ala Lys Leu
               500                 505
<210> 4
   <211> 532
   <212> PRT
   <213> Homo sapiens
<400> 4
   Met Ala Pro Ser Ser Pro Arg Pro Ala Leu Pro Ala Leu Leu Val Leu
   1               5                   10                  15
   Leu Gly Ala Leu Phe Pro Gly Pro Gly Asn Ala Gln Thr Ser Val Ser
               20                  25                  30
   Pro Ser Lys Val Ile Leu Pro Arg Gly Gly Ser Val Leu Val Thr Cys
           35                  40                  45
   Ser Thr Ser Cys Asp Gln Pro Lys Leu Leu Gly Ile Glu Thr Pro Leu
       50                  55                  60
   Pro Lys Lys Glu Leu Leu Leu Pro Gly Asn Asn Arg Lys Val Tyr Glu
   65                  70                  75                  80
   Leu Ser Asn Val Gln Glu Asp Ser Gln Pro Met Cys Tyr Ser Asn Cys
                   85                  90                  95
   Pro Asp Gly Gln Ser Thr Ala Lys Thr Phe Leu Thr Val Tyr Trp Thr
               100                 105                 110
   Pro Glu Arg Val Glu Leu Ala Pro Leu Pro Ser Trp Gln Pro Val Gly
           115                 120                 125
   Lys Asn Leu Thr Leu Arg Cys Gln Val Glu Gly Gly Ala Pro Arg Ala
       130                 135                 140
   Asn Leu Thr Val Val Leu Leu Arg Gly Glu Lys Glu Leu Lys Arg Glu
   145                 150                 155                 160
   Pro Ala Val Gly Glu Pro Ala Glu Val Thr Thr Thr Val Leu Val Arg
                   165                 170                 175
   Arg Asp His His Gly Ala Asn Phe Ser Cys Arg Thr Glu Leu Asp Leu
   180 185 190
   Arg Pro Gln Gly Leu Glu Leu Phe Glu Asn Thr Ser Ala Pro Tyr Gln
   195 200 205
   Leu Gln Thr Phe Val Leu Pro Ala Thr Pro Pro Gln Leu Val Ser Pro
   210 215 220
   Arg Val Leu Glu Val Asp Thr Gln Gly Thr Val Val Cys Ser Leu Asp
   225 230 235 240
   Gly Leu Phe Pro Val Ser Glu Ala Gln Val His Leu Ala Leu Gly Asp
   245 250 255
   Gln Arg Leu Asn Pro Thr Val Thr Tyr Gly Asn Asp Ser Phe Ser Ala
   260 265 270
   Lys Ala Ser Val Ser Val Thr Ala Glu Asp Glu Gly Thr Gln Arg Leu
   275 280 285
   Thr Cys Ala Val Ile Leu Gly Asn Gln Ser Gln Glu Thr Leu Gln Thr
   290 295 300
   Val Thr Ile Tyr Ser Phe Pro Ala Pro Asn Val Ile Leu Thr Lys Pro
   305 310 315 320
   Glu Val Ser Glu Gly Thr Glu Val Thr Val Lys Cys Glu Ala His Pro
   325 330 335
   Arg Ala Lys Val Thr Leu Asn Gly Val Pro Ala Gln Pro Leu Gly Pro
   340 345 350
   Arg Ala Gln Leu Leu Leu Lys Ala Thr Pro Glu Asp Asn Gly Arg Ser
   355 360 365
   Phe Ser Cys Ser Ala Thr Leu Glu Val Ala Gly Gln Leu Ile His Lys
   370 375 380
   Asn Gln Thr Arg Glu Leu Arg Val Leu Tyr Gly Pro Arg Leu Asp Glu
   385 390 395 400
   Arg Asp Cys Pro Gly Asn Trp Thr Trp Pro Glu Asn Ser Gln Gln Thr
   405 410 415
   Pro Met Cys Gln Ala Trp Gly Asn Pro Leu Pro Glu Leu Lys Cys Leu
   420 425 430
   Lys Asp Gly Thr Phe Pro Leu Pro Ile Gly Glu Ser Val Thr Val Thr
   435 440 445
   Arg Asp Leu Glu Gly Thr Tyr Leu Cys Arg Ala Arg Ser Thr Gln Gly
   450 455 460
   Glu Val Thr Arg Lys Val Thr Val Asn Val Leu Ser Pro Arg Tyr Glu
   465 470 475 480
   Ile Val Ile Ile Thr Val Val Ala Ala Ala Val Ile Met Gly Thr Ala
   485 490 495
   Gly Leu Ser Thr Tyr Leu Tyr Asn Arg Gln Arg Lys Ile Lys Lys Tyr
   500 505 510
   Arg Leu Gln Gln Ala Gln Lys Gly Thr Pro Met Lys Pro Asn Thr Gln
   515 520 525
   Ala Thr Pro Pro
   530
<210> 5
   <211> 628
   <212> PRT
   <213> Homo sapiens
<400> 5
   Met Glu Pro Pro Asp Ala Pro Ala Gln Ala Arg Gly Ala Pro Arg Leu
   1               5                   10                  15
   Leu Leu Leu Ala Val Leu Leu Ala Ala His Pro Asp Ala Gln Ala Glu
               20                  25                  30
   Val Arg Leu Ser Val Pro Pro Leu Val Glu Val Met Arg Gly Lys Ser
           35                  40                  45
   Val Ile Leu Asp Cys Thr Pro Thr Gly Thr His Asp His Tyr Met Leu
       50                  55                  60
   Glu Trp Phe Leu Thr Asp Arg Ser Gly Ala Arg Pro Arg Leu Ala Ser
   65                  70                  75                  80
   Ala Glu Met Gln Gly Ser Glu Leu Gln Val Thr Met His Asp Thr Arg
                   85                  90                  95
   Gly Arg Ser Pro Pro Tyr Gln Leu Asp Ser Gln Gly Arg Leu Val Leu
               100                 105                 110
   Ala Glu Ala Gln Val Gly Asp Glu Arg Asp Tyr Val Cys Val Val Arg
           115                 120                 125
   Ala Gly Ala Ala Gly Thr Ala Glu Ala Thr Ala Arg Leu Asn Val Phe
   130 135 140
   Ala Lys Pro Glu Ala Thr Glu Val Ser Pro Asn Lys Gly Thr Leu Ser
   145 150 155 160
   Val Met Glu Asp Ser Ala Gln Glu Ile Ala Thr Cys Asn Ser Arg Asn
   165 170 175
   Gly Asn Pro Ala Pro Lys Ile Thr Trp Tyr Arg Asn Gly Gln Arg Leu
   180 185 190
   Glu Val Pro Val Glu Met Asn Pro Glu Gly Tyr Met Thr Ser Arg Thr
   195 200 205
   Val Arg Glu Ala Ser Gly Leu Leu Ser Leu Thr Ser Thr Leu Tyr Leu
   210 215 220
   Arg Leu Arg Lys Asp Asp Arg Asp Ala Ser Phe His Cys Ala Ala His
   225 230 235 240
   Tyr Ser Leu Pro Glu Gly Arg His Gly Arg Leu Asp Ser Pro Thr Phe
   245 250 255
   His Leu Thr Leu His Tyr Pro Thr Glu His Val Gln Phe Trp Val Gly
   260 265 270
   Ser Pro Ser Thr Pro Ala Gly Trp Val Arg Glu Gly Asp Thr Val Gln
   275 280 285
   Leu Leu Cys Arg Gly Asp Gly Ser Pro Ser Pro Glu Tyr Thr Leu Phe
   290 295 300
   Arg Leu Gln Asp Glu Gln Glu Glu Val Leu Asn Val Asn Leu Glu Gly
   305 310 315 320
   Asn Leu Thr Leu Glu Gly Val Thr Arg Gly Gln Ser Gly Thr Tyr Gly
   325 330 335
   Cys Arg Val Glu Asp Tyr Asp Ala Ala Asp Asp Val Gln Leu Ser Lys
   340 345 350
   Thr Leu Glu Leu Arg Val Ala Tyr Leu Asp Pro Leu Glu Leu Ser Glu
   355 360 365
   Gly Lys Val Leu Ser Leu Pro Leu Asn Ser Ser Ala Val Val Asn Cys
   370 375 380
   Ser Val His Gly Leu Pro Thr Pro Ala Leu Arg Trp Thr Lys Asp Ser
   385 390 395 400
   Thr Pro Leu Gly Asp Gly Pro Met Leu Ser Leu Ser Ser Ile Thr Phe
   405 410 415
   Asp Ser Asn Gly Thr Tyr Val Cys Glu Ala Ser Leu Pro Thr Val Pro
   420 425 430
   Val Leu Ser Arg Thr Gln Asn Phe Thr Leu Leu Val Gln Gly Ser Pro
   435 440 445
   Glu Leu Lys Thr Ala Glu Ile Glu Pro Lys Ala Asp Gly Ser Trp Arg
   450 455 460
   Glu Gly Asp Glu Val Thr Leu Ile Cys Ser Ala Arg Gly His Pro Asp
   465 470 475 480
   Pro Lys Leu Ser Trp Ser Gln Leu Gly Gly Ser Pro Ala Glu Pro Ile
   485 490 495
   Pro Gly Arg Gln Gly Trp Val Ser Ser Ser Leu Thr Leu Lys Val Thr
   500 505 510
   Ser Ala Leu Ser Arg Asp Gly Ile Ser Cys Glu Ala Ser Asn Pro His
   515 520 525
   Gly Asn Lys Arg His Val Phe His Phe Gly Thr Val Ser Pro Gln Thr
   530 535 540
   Ser Gln Ala Gly Val Ala Val Met Ala Val Ala Val Ser Val Gly Leu
   545 550 555 560
   Leu Leu Leu Val Val Ala Val Phe Tyr Cys Val Arg Arg Lys Gly Gly
   565 570 575
   Pro Cys Cys Arg Gln Arg Arg Glu Lys Gly Ala Pro Pro Pro Gly Glu
   580 585 590
   Pro Gly Leu Ser His Ser Gly Ser Glu Gln Pro Glu Gln Thr Gly Leu
   595 600 605
   Leu Met Gly Gly Ala Ser Gly Gly Ala Arg Gly Gly Ser Gly Gly Phe
   610 615 620
   Gly Asp Glu Cys
   625
<210> 6
   <211> 253
   <212> PRT
   <213> Homo sapiens
<400> 6
<210> 7
   <211> 895
   <212> PRT
   <213> Homo sapiens
<400> 7
   Met Arg Met Ser Val Gly Leu Ser Leu Leu Leu Pro Leu Ser Gly Arg
   1               5                   10                  15
   Thr Phe Leu Leu Leu Leu Ser Val Val Met Ala Gln Ser His Trp Pro
               20                  25                  30
   Ser Glu Pro Ser Glu Ala Val Arg Asp Trp Glu Asn Gln Leu Glu Ala
           35                  40                  45
   Ser Met His Ser Val Leu Ser Asp Leu His Glu Ala Val Pro Thr Val
       50                  55                  60
   Val Gly Ile Pro Asp Gly Thr Ala Val Val Gly Arg Ser Phe Arg Val
   65                  70                  75                  80
   Thr Ile Pro Thr Asp Leu Ile Ala Ser Ser Gly Asp Ile Ile Lys Val
                   85                  90                  95
   Ser Ala Ala Gly Lys Glu Ala Leu Pro Ser Trp Leu His Trp Asp Ser
               100                 105                 110
   Gln Ser His Thr Leu Glu Gly Leu Pro Leu Asp Thr Asp Lys Gly Val
           115                 120                 125
   His Tyr Ile Ser Val Ser Ala Thr Arg Leu Gly Ala Asn Gly Ser His
       130                 135                 140
   Ile Pro Gln Thr Ser Ser Val Phe Ser Ile Glu Val Tyr Pro Glu Asp
   145                 150                 155                 160
   His Ser Glu Leu Gln Ser Val Arg Thr Ala Ser Pro Asp Pro Gly Glu
                   165                 170                 175
   Val Val Ser Ser Ala Cys Ala Ala Asp Glu Pro Val Thr Val Leu Thr
               180                 185                 190
   Val Ile Leu Asp Ala Asp Leu Thr Lys Met Thr Pro Lys Gln Arg Ile
           195                 200                 205
   Asp Leu Leu His Arg Met Arg Ser Phe Ser Glu Val Glu Leu His Asn
   210 215 220
   Met Lys Leu Val Pro Val Val Asn Asn Arg Leu Phe Asp Met Ser Ala
   225 230 235 240
   Phe Met Ala Gly Pro Gly Asn Ala Lys Lys Val Val Glu Asn Gly Ala
   245 250 255
   Leu Leu Ser Trp Lys Leu Gly Cys Ser Leu Asn Gln Asn Ser Val Pro
   260 265 270
   Asp Ile His Gly Val Glu Ala Pro Ala Arg Glu Gly Ala Met Ser Ala
   275 280 285
   Gln Leu Gly Tyr Pro Val Val Gly Trp His Ile Ala Asn Lys Lys Pro
   290 295 300
   Pro Leu Pro Lys Arg Val Arg Arg Gln Ile His Ala Thr Pro Thr Pro
   305 310 315 320
   Val Thr Ala Ile Gly Pro Pro Thr Thr Ala Ile Gln Glu Pro Pro Ser
   325 330 335
   Arg Ile Val Pro Thr Pro Thr Ser Pro Ala Ile Ala Pro Pro Thr Glu
   340 345 350
   Thr Met Ala Pro Pro Val Arg Asp Pro Val Pro Gly Lys Pro Thr Val
   355 360 365
   Thr Ile Arg Thr Arg Gly Ala Ile Ile Gln Thr Pro Thr Leu Gly Pro
   370 375 380
   Ile Gln Pro Thr Arg Val Ser Glu Ala Gly Thr Thr Val Pro Gly Gln
   385 390 395 400
   Ile Arg Pro Thr Met Thr Ile Pro Gly Tyr Val Glu Pro Thr Ala Val
   405 410 415
   Ala Thr Pro Pro Thr Thr Thr Thr Lys Lys Pro Arg Val Ser Thr Pro
   420 425 430
   Lys Pro Ala Thr Pro Ser Thr Asp Ser Thr Thr Thr Thr Thr Arg Arg
   435 440 445
   Pro Thr Lys Lys Pro Arg Thr Pro Arg Pro Val Pro Arg Val Thr Thr
   450 455 460
   Lys Val Ser Ile Thr Arg Leu Glu Thr Ala Ser Pro Pro Thr Arg Ile
   465 470 475 480
   Arg Thr Thr Thr Ser Gly Val Pro Arg Gly Gly Glu Pro Asn Gln Arg
   485 490 495
   Pro Glu Leu Lys Asn His Ile Asp Arg Val Asp Ala Trp Val Gly Thr
   500 505 510
   Tyr Phe Glu Val Lys Ile Pro Ser Asp Thr Phe Tyr Asp His Glu Asp
   515 520 525
   Thr Thr Thr Asp Lys Leu Lys Leu Thr Leu Lys Leu Arg Glu Gln Gln
   530 535 540
   Leu Val Gly Glu Lys Ser Trp Val Gln Phe Asn Ser Asn Ser Gln Leu
   545 550 555 560
   Met Tyr Gly Leu Pro Asp Ser Ser His Val Gly Lys His Glu Tyr Phe
   565 570 575
   Met His Ala Thr Asp Lys Gly Gly Leu Ser Ala Val Asp Ala Phe Glu
   580 585 590
   Ile His Val His Arg Arg Pro Gln Gly Asp Arg Ala Pro Ala Arg Phe
   595 600 605
   Lys Ala Lys Phe Val Gly Asp Pro Ala Leu Val Leu Asn Asp Ile His
   610 615 620
   Lys Lys Ile Ala Leu Val Lys Lys Leu Ala Phe Ala Phe Gly Asp Arg
   625 630 635 640
   Asn Cys Ser Thr Ile Thr Leu Gln Asn Ile Thr Arg Gly Ser Ile Val
   645 650 655
   Val Glu Trp Thr Asn Asn Thr Leu Pro Leu Glu Pro Cys Pro Lys Glu
   660 665 670
   Gln Ile Ala Gly Leu Ser Arg Arg Ile Ala Glu Asp Asp Gly Lys Pro
   675 680 685
   Arg Pro Ala Phe Ser Asn Ala Leu Glu Pro Asp Phe Lys Ala Thr Ser
   690 695 700
   Ile Thr Val Thr Gly Ser Gly Ser Cys Arg His Leu Gln Phe Ile Pro
   705 710 715 720
   Val Val Pro Pro Arg Arg Val Pro Ser Glu Ala Pro Pro Thr Glu Val
   725 730 735
   Pro Asp Arg Asp Pro Glu Lys Ser Ser Glu Asp Asp Val Tyr Leu His
   740 745 750
   Thr Val Ile Pro Ala Val Val Val Ala Ala Ile Leu Leu Ile Ala Gly
   755 760 765
   Ile Ile Ala Met Ile Cys Tyr Arg Lys Lys Arg Lys Gly Lys Leu Thr
   770 775 780
   Leu Glu Asp Gln Ala Thr Phe Ile Lys Lys Gly Val Pro Ile Ile Phe
   785 790 795 800
   Ala Asp Glu Leu Asp Asp Ser Lys Pro Pro Pro Ser Ser Ser Met Pro
   805 810 815
   Leu Ile Leu Gln Glu Glu Lys Ala Pro Leu Pro Pro Pro Glu Tyr Pro
   820 825 830
   Asn Gln Ser Val Pro Glu Thr Thr Pro Leu Asn Gln Asp Thr Met Gly
   835 840 845
   Glu Tyr Thr Pro Leu Arg Asp Glu Asp Pro Asn Ala Pro Pro Tyr Gln
   850 855 860
   Pro Pro Pro Pro Phe Thr Ala Pro Met Glu Gly Lys Gly Ser Arg Pro
   865 870 875 880
   Lys Asn Met Thr Pro Tyr Arg Ser Pro Pro Pro Tyr Val Pro Pro
   885 890 895
<210> 8
   <211> 926
   <212> PRT
   <213> Homo sapiens
<400> 8
   Ser Ala Arg Glu Glu Ser Gly Ala Gly Ala Arg Pro Arg Arg Arg Ser
   1               5                   10                  15
   Ala Asp Ser Gly Ala Ala Gly Ala Gly Arg Gly Gly Gly Gly Glu Ala
               20                  25                  30
   Ala Gly Lys Glu Glu Glu Gly Glu Ser Arg Ser Arg Arg Ala Ser Met
           35                  40                  45
   Gly Arg Leu Ala Ser Arg Pro Leu Leu Leu Ala Leu Leu Ser Leu Ala
       50                  55                  60
   Leu Cys Arg Gly Arg Val Val Arg Val Pro Thr Ala Thr Leu Val Arg
   65 70 75 80
   Val Val Gly Thr Glu Leu Val Ile Pro Cys Asn Val Ser Asp Tyr Asp
   85 90 95
   Gly Pro Ser Glu Gln Asn Phe Asp Trp Ser Phe Ser Ser Leu Gly Ser
   100 105 110
   Ser Phe Val Glu Leu Ala Ser Thr Trp Glu Val Gly Phe Pro Ala Gln
   115 120 125
   Leu Tyr Gln Glu Arg Leu Gln Arg Gly Glu Ile Leu Leu Arg Arg Thr
   130 135 140
   Ala Asn Asp Ala Val Glu Leu His Ile Lys Asn Val Gln Pro Ser Asp
   145 150 155 160
   Gln Gly His Tyr Lys Cys Ser Thr Pro Ser Thr Asp Ala Thr Val Gln
   165 170 175
   Gly Asn Tyr Glu Asp Thr Val Gln Val Lys Val Leu Ala Asp Ser Leu
   180 185 190
   His Val Gly Pro Ser Ala Arg Pro Pro Pro Ser Leu Ser Leu Arg Glu
   195 200 205
   Gly Glu Pro Phe Glu Leu Arg Cys Thr Ala Ala Ser Ala Ser Pro Leu
   210 215 220
   His Thr His Leu Ala Leu Leu Trp Glu Val His Arg Gly Pro Ala Arg
   225 230 235 240
   Arg Ser Val Leu Ala Leu Thr His Glu Gly Arg Phe His Pro Gly Leu
   245 250 255
   Gly Tyr Glu Gln Arg Tyr His Ser Gly Asp Val Arg Leu Asp Thr Val
   260 265 270
   Gly Ser Asp Ala Tyr Arg Leu Ser Val Ser Arg Ala Leu Ser Ala Asp
   275 280 285
   Gln Gly Ser Tyr Arg Cys Ile Val Ser Glu Trp Ile Ala Glu Gln Gly
   290 295 300
   Asn Trp Gln Glu Ile Gln Glu Lys Ala Val Glu Val Ala Thr Val Val
   305 310 315 320
   Ile Gln Pro Ser Val Leu Arg Ala Ala Val Pro Lys Asn Val Ser Val
   325 330 335
   Ala Glu Gly Lys Glu Leu Asp Leu Thr Cys Asn Ile Thr Thr Asp Arg
               340                 345                 350
   Ala Asp Asp Val Arg Pro Glu Val Thr Trp Ser Phe Ser Arg Met Pro
           355                 360                 365
   Asp Ser Thr Leu Pro Gly Ser Arg Val Leu Ala Arg Leu Asp Arg Asp
       370                 375                 380
   Ser Leu Val His Ser Ser Pro His Val Ala Leu Ser His Val Asp Ala
   385                 390                 395                 400
   Arg Ser Tyr His Leu Leu Val Arg Asp Val Ser Lys Glu Asn Ser Gly
                   405                 410                 415
   Tyr Tyr Tyr Cys His Val Ser Leu Trp Ala Pro Gly His Asn Arg Ser
               420                 425                 430
   Trp His Lys Val Ala Glu Ala Val Ser Ser Pro Ala Gly Val Gly Val
           435                 440                 445
   Thr Trp Leu Glu Pro Asp Tyr Gln Val Tyr Leu Asn Ala Ser Lys Val
       450                 455                 460
   Pro Gly Phe Ala Asp Asp Pro Thr Glu Leu Ala Cys Arg Val Val Asp
   465                 470                 475                 480
   Thr Lys Ser Gly Glu Ala Asn Val Arg Phe Thr Val Ser Trp Tyr Tyr
                   485                 490                 495
   Arg Met Asn Arg Arg Ser Asp Asn Val Val Thr Ser Glu Leu Leu Ala
               500                 505                 510
   Val Met Asp Gly Asp Trp Thr Leu Lys Tyr Gly Glu Arg Ser Lys Gln
           515                 520                 525
   Arg Ala Gln Asp Gly Asp Phe Ile Phe Ser Lys Glu His Thr Asp Thr
       530                 535                 540
   Phe Asn Phe Arg Ile Gln Arg Thr Thr Glu Glu Asp Arg Gly Asn Tyr
   545                 550                 555                 560
   Tyr Cys Val Val Ser Ala Trp Thr Lys Gln Arg Asn Asn Ser Trp Val
                   565                 570                 575
   Lys Ser Lys Asp Val Phe Ser Lys Pro Val Asn Ile Phe Trp Ala Leu
               580                 585                 590
   Glu Asp Ser Val Leu Val Val Lys Ala Arg Gln Pro Lys Pro Phe Phe
   595 600 605
   Ala Ala Gly Asn Thr Phe Glu Met Thr Cys Lys Val Ser Ser Lys Asn
   610 615 620
   Ile Lys Ser Pro Arg Tyr Ser Val Leu Ile Met Ala Glu Lys Pro Val
   625 630 635 640
   Gly Asp Leu Ser Ser Pro Asn Glu Thr Lys Tyr Ile Ile Ser Leu Asp
   645 650 655
   Gln Asp Ser Val Val Lys Leu Glu Asn Trp Thr Asp Ala Ser Arg Val
   660 665 670
   Asp Gly Val Val Leu Glu Lys Val Gln Glu Asp Glu Phe Arg Tyr Arg
   675 680 685
   Met Tyr Gln Thr Gln Val Ser Asp Ala Gly Leu Tyr Arg Cys Met Val
   690 695 700
   Thr Ala Trp Ser Pro Val Arg Gly Ser Leu Trp Arg Glu Ala Ala Thr
   705 710 715 720
   Ser Leu Ser Asn Pro Ile Glu Ile Asp Phe Gln Thr Ser Gly Pro Ile
   725 730 735
   Phe Asn Ala Ser Val His Ser Asp Thr Pro Ser Val Ile Arg Gly Asp
   740 745 750
   Leu Ile Lys Leu Phe Cys Ile Ile Thr Val Glu Gly Ala Ala Leu Asp
   755 760 765
   Pro Asp Asp Met Ala Phe Asp Val Ser Trp Phe Ala Val His Ser Phe
   770 775 780
   Gly Leu Asp Lys Ala Pro Val Leu Leu Ser Ser Leu Asp Arg Lys Gly
   785 790 795 800
   Ile Val Thr Thr Ser Arg Arg Asp Trp Lys Ser Asp Leu Ser Leu Glu
   805 810 815
   Arg Val Ser Val Leu Glu Phe Leu Leu Gln Val His Gly Ser Glu Asp
   820 825 830
   Gln Asp Phe Gly Asn Tyr Tyr Cys Ser Val Thr Pro Trp Val Lys Ser
   835 840 845
   Pro Thr Gly Ser Trp Gln Lys Glu Ala Glu Ile His Ser Lys Pro Val
   850 855 860
   Phe Ile Thr Val Lys Met Asp Val Leu Asn Ala Phe Lys Tyr Pro Leu
   865 870 875 880
   Leu Ile Gly Val Gly Leu Ser Thr Val Ile Gly Leu Leu Ser Cys Leu
   885 890 895
   Ile Gly Tyr Cys Ser Ser His Trp Cys Cys Lys Lys Glu Val Gln Glu
   900 905 910
   Thr Arg Arg Glu Arg Arg Arg Leu Met Ser Met Glu Met Asp
   915 920 925
<210> 9
   <211> 613
   <212> PRT
   <213> Homo sapiens
<400> 9
   Met Gly Ala Leu Arg Pro Thr Leu Leu Pro Pro Ser Leu Pro Leu Leu
   1 5 10 15
   Leu Leu Leu Met Leu Gly Met Gly Cys Trp Ala Arg Glu Val Leu Val
   20 25 30
   Pro Glu Gly Pro Leu Tyr Arg Val Ala Gly Thr Ala Val Ser Ile Ser
   35 40 45
   Cys Asn Val Thr Gly Tyr Glu Gly Pro Ala Gln Gln Asn Phe Glu Trp
   50 55 60
   Phe Leu Tyr Arg Pro Glu Ala Pro Asp Thr Ala Leu Gly Ile Val Ser
   65 70 75 80
   Thr Lys Asp Thr Gln Phe Ser Tyr Ala Val Phe Lys Ser Arg Val Val
   85 90 95
   Ala Gly Glu Val Gln Val Gln Arg Leu Gln Gly Asp Ala Val Val Leu
   100 105 110
   Lys Ile Ala Arg Leu Gln Ala Gln Asp Ala Gly Ile Tyr Glu Cys His
   115 120 125
   Thr Pro Ser Thr Asp Thr Arg Tyr Leu Gly Ser Tyr Ser Gly Lys Val
   130 135 140
   Glu Leu Arg Val Leu Pro Asp Val Leu Gln Val Ser Ala Ala Pro Pro
   145 150 155 160
   Gly Pro Arg Gly Arg Gln Ala Pro Thr Ser Pro Pro Arg Met Thr Val
   165 170 175
   His Glu Gly Gln Glu Leu Ala Leu Gly Cys Leu Ala Arg Thr Ser Thr
   180 185 190
   Gln Lys His Thr His Leu Ala Val Ser Phe Gly Arg Ser Val Pro Glu
   195 200 205
   Ala Pro Val Gly Arg Ser Thr Leu Gln Glu Val Val Gly Ile Arg Ser
   210 215 220
   Asp Leu Ala Val Glu Ala Gly Ala Pro Tyr Ala Glu Arg Leu Ala Ala
   225 230 235 240
   Gly Glu Leu Arg Leu Gly Lys Glu Gly Thr Asp Arg Tyr Arg Met Val
   245 250 255
   Val Gly Gly Ala Gln Ala Gly Asp Ala Gly Thr Tyr His Cys Thr Ala
   260 265 270
   Ala Glu Trp Ile Gln Asp Pro Asp Gly Ser Trp Ala Gln Ile Ala Glu
   275 280 285
   Lys Arg Ala Val Leu Ala His Val Asp Val Gln Thr Leu Ser Ser Gln
   290 295 300
   Leu Ala Val Thr Val Gly Pro Gly Glu Arg Arg Ile Gly Pro Gly Glu
   305 310 315 320
   Pro Leu Glu Leu Leu Cys Asn Val Ser Gly Ala Leu Pro Pro Ala Gly
   325 330 335
   Arg His Ala Ala Tyr Ser Val Gly Trp Glu Met Ala Pro Ala Gly Ala
   340 345 350
   Pro Gly Pro Gly Arg Leu Val Ala Gln Leu Asp Thr Glu Gly Val Gly
   355 360 365
   Ser Leu Gly Pro Gly Tyr Glu Gly Arg His Ile Ala Met Glu Lys Val
   370 375 380
   Ala Ser Arg Thr Tyr Arg Leu Arg Leu Glu Ala Ala Arg Pro Gly Asp
   385 390 395 400
   Ala Gly Thr Tyr Arg Cys Leu Ala Lys Ala Tyr Val Arg Gly Ser Gly
   405 410 415
   Thr Arg Leu Arg Glu Ala Ala Ser Ala Arg Ser Arg Pro Leu Pro Val
   420 425 430
   His Val Arg Glu Glu Gly Val Val Leu Glu Ala Val Ala Trp Leu Ala
   435 440 445
   Gly Gly Thr Val Tyr Arg Gly Glu Thr Ala Ser Leu Leu Cys Asn Ile
   450 455 460
   Ser Val Arg Gly Gly Pro Pro Gly Leu Arg Leu Ala Ala Ser Trp Trp
   465 470 475 480
   Val Glu Arg Pro Glu Asp Gly Glu Leu Ser Ser Val Pro Ala Gln Leu
   485 490 495
   Val Gly Gly Val Gly Gln Asp Gly Val Ala Glu Leu Gly Val Arg Pro
   500 505 510
   Gly Gly Gly Pro Val Ser Val Glu Leu Val Gly Pro Arg Ser His Arg
   515 520 525
   Leu Arg Leu His Ser Leu Gly Pro Glu Asp Glu Gly Val Tyr His Cys
   530 535 540
   Ala Pro Ser Ala Trp Val Gln His Ala Asp Tyr Ser Trp Tyr Gln Ala
   545 550 555 560
   Gly Ser Ala Arg Ser Gly Pro Val Thr Val Tyr Pro Tyr Met His Ala
   565 570 575
   Leu Asp Thr Leu Phe Val Pro Leu Leu Val Gly Thr Gly Val Ala Leu
   580 585 590
   Val Thr Gly Ala Thr Val Leu Gly Thr Ile Thr Cys Cys Phe Met Lys
   595 600 605
   Arg Leu Arg Lys Arg
   610
<210> 10
   <211> 860
   <212> PRT
   <213> Homo sapiens
<400> 10
<210> 11
   <211> 542
   <212> PRT
   <213> Homo sapiens
<400> 11
   Met Thr Lys Ser Asn Gly Glu Glu Pro Lys Met Gly Gly Arg Met Glu
   1               5                   10                  15
   Arg Phe Gln Gln Gly Val Arg Lys Arg Thr Leu Leu Ala Lys Lys Lys
               20                  25                  30
   Val Gln Asn Ile Thr Lys Glu Asp Val Lys Ser Tyr Leu Phe Arg Asn
           35                  40                  45
   Ala Phe Val Leu Leu Thr Val Thr Ala Val Ile Val Gly Thr Ile Leu
       50                  55                  60
   Gly Phe Thr Leu Arg Pro Tyr Arg Met Ser Tyr Arg Glu Val Lys Tyr
   65                  70                  75                  80
   Phe Ser Phe Pro Gly Glu Leu Leu Met Arg Met Leu Gln Met Leu Val
                   85                  90                  95
   Leu Pro Leu Ile Ile Ser Ser Leu Val Thr Gly Met Ala Ala Leu Asp
               100                 105                 110
   Ser Lys Ala Ser Gly Lys Met Gly Met Arg Ala Val Val Tyr Tyr Met
           115                 120                 125
   Thr Thr Thr Ile Ile Ala Val Val Ile Gly Ile Ile Ile Val Ile Ile
       130                 135                 140
   Ile His Pro Gly Lys Gly Thr Lys Glu Asn Met His Arg Glu Gly Lys
   145                 150                 155                 160
   Ile Val Arg Val Thr Ala Ala Asp Ala Phe Leu Asp Leu Ile Arg Asn
                   165                 170                 175
   Met Phe Pro Pro Asn Leu Val Glu Ala Cys Phe Lys Gln Phe Lys Thr
               180                 185                 190
   Asn Tyr Glu Lys Arg Ser Phe Lys Val Pro Ile Gln Ala Asn Glu Thr
           195                 200                 205
   Leu Val Gly Ala Val Ile Asn Asn Val Ser Glu Ala Met Glu Thr Leu
       210                 215                 220
   Thr Arg Ile Thr Glu Glu Leu Val Pro Val Pro Gly Ser Val Asn Gly
   225                 230                 235                 240
   Val Asn Ala Leu Gly Leu Val Val Phe Ser Met Cys Phe Gly Phe Val
                   245                 250                 255
   Ile Gly Asn Met Lys Glu Gln Gly Gln Ala Leu Arg Glu Phe Phe Asp
               260                 265                 270
   Ser Leu Asn Glu Ala Ile Met Arg Leu Val Ala Val Ile Met Trp Tyr
           275                 280                 285
   Ala Pro Val Gly Ile Leu Phe Leu Ile Ala Gly Lys Ile Val Glu Met
       290                 295                 300
   Glu Asp Met Gly Val Ile Gly Gly Gln Leu Ala Met Tyr Thr Val Thr
   305                 310                 315                 320
   Val Ile Val Gly Leu Leu Ile His Ala Val Ile Val Leu Pro Leu Leu
                   325                 330                 335
   Tyr Phe Leu Val Thr Arg Lys Asn Pro Trp Val Phe Ile Gly Gly Leu
               340                 345                 350
   Leu Gln Ala Leu Ile Thr Ala Leu Gly Thr Ser Ser Ser Ser Ala Thr
           355                 360                 365
   Leu Pro Ile Thr Phe Lys Cys Leu Glu Glu Asn Asn Gly Val Asp Lys
       370                 375                 380
   Arg Val Thr Arg Phe Val Leu Pro Val Gly Ala Thr Ile Asn Met Asp
   385                 390                 395                 400
   Gly Thr Ala Leu Tyr Glu Ala Leu Ala Ala Ile Phe Ile Ala Gln Val
                   405                 410                 415
   Asn Asn Phe Glu Leu Asn Phe Gly Gln Ile Ile Thr Ile Ser Ile Thr
               420                 425                 430
   Ala Thr Ala Ala Ser Ile Gly Ala Ala Gly Ile Pro Gln Ala Gly Leu
           435                     440                     445
   Val Thr Met Val Ile Val Leu Thr Ser Val Gly Leu Pro Thr Asp Asp
       450                     455                     460
   Ile Thr Leu Ile Ile Ala Val Asp Trp Phe Leu Asp Arg Leu Arg Thr
   465                     470                     475                     480
   Thr Thr Asn Val Leu Gly Asp Ser Leu Gly Ala Gly Ile Val Glu His
                   485                     490                     495
   Leu Ser Arg His Glu Leu Lys Asn Arg Asp Val Glu Met Gly Asn Ser
               500                     505                     510
   Val Ile Glu Glu Asn Glu Met Lys Lys Pro Tyr Gln Leu Ile Ala Gln
           515                     520                     525
   Asp Asn Glu Thr Glu Lys Pro Ile Asp Ser Glu Thr Lys Met
       530                     535                     540
<210> 12
   <211> 698
   <212> PRT
   <213> Homo sapiens
<400> 12
   Met Gln Ala His Glu Leu Phe Arg Tyr Phe Arg Met Pro Glu Leu Val
   1               5                   10                  15
   Asp Phe Arg Gln Tyr Val Arg Thr Leu Pro Thr Asn Thr Leu Met Gly
               20                  25                  30
   Phe Gly Ala Phe Ala Ala Leu Thr Thr Phe Trp Tyr Ala Thr Arg Pro
           35                  40                  45
   Lys Pro Leu Lys Pro Pro Cys Asp Leu Ser Met Gln Ser Val Glu Val
       50                  55                  60
   Ala Gly Ser Gly Gly Ala Arg Arg Ser Ala Leu Leu Asp Ser Asp Glu
   65                  70                  75                  80
   Pro Leu Val Tyr Phe Tyr Asp Asp Val Thr Thr Leu Tyr Glu Gly Phe
                   85                  90                  95
   Gln Arg Gly Ile Gln Val Ser Asn Asn Gly Pro Cys Leu Gly Ser Arg
               100                 105                 110
   Lys Pro Asp Gln Pro Tyr Glu Trp Leu Ser Tyr Lys Gln Val Ala Glu
   115 120 125
   Leu Ser Glu Cys Ile Gly Ser Ala Leu Ile Gln Lys Gly Phe Lys Thr
   130 135 140
   Ala Pro Asp Gln Phe Ile Gly Ile Phe Ala Gln Asn Arg Pro Glu Trp
   145 150 155 160
   Val Ile Ile Glu Gln Gly Cys Phe Ala Tyr Ser Met Val Ile Val Pro
   165 170 175
   Leu Tyr Asp Thr Leu Gly Asn Glu Ala Ile Thr Tyr Ile Val Asn Lys
   180 185 190
   Ala Glu Leu Ser Leu Val Phe Val Asp Lys Pro Glu Lys Ala Lys Leu
   195 200 205
   Leu Leu Glu Gly Val Glu Asn Lys Leu Ile Pro Gly Leu Lys Ile Ile
   210 215 220
   Val Val Met Asp Ala Tyr Gly Ser Glu Leu Val Glu Arg Gly Gln Arg
   225 230 235 240
   Cys Gly Val Glu Val Thr Ser Met Lys Ala Met Glu Asp Leu Gly Arg
   245 250 255
   Ala Asn Arg Arg Lys Pro Lys Pro Pro Ala Pro Glu Asp Leu Ala Val
   260 265 270
   Ile Cys Phe Thr Ser Gly Thr Thr Gly Asn Pro Lys Gly Ala Met Val
   275 280 285
   Thr His Arg Asn Ile Val Ser Asp Cys Ser Ala Phe Val Lys Ala Thr
   290 295 300
   Glu Asn Thr Val Asn Pro Cys Pro Asp Asp Thr Leu Ile Ser Phe Leu
   305 310 315 320
   Pro Leu Ala His Met Phe Glu Arg Val Val Glu Cys Val Met Leu Cys
   325 330 335
   His Gly Ala Lys Ile Gly Phe Phe Gln Gly Asp Ile Arg Leu Leu Met
   340 345 350
   Asp Asp Leu Lys Val Leu Gln Pro Thr Val Phe Pro Val Val Pro Arg
   355 360 365
   Leu Leu Asn Arg Met Phe Asp Arg Ile Phe Gly Gln Ala Asn Thr Thr
   370 375 380
   Leu Lys Arg Trp Leu Leu Asp Phe Ala Ser Lys Arg Lys Glu Ala Glu
   385 390 395 400
   Leu Arg Ser Gly Ile Ile Arg Asn Asn Ser Leu Trp Asp Arg Leu Ile
   405 410 415
   Phe His Lys Val Gln Ser Ser Leu Gly Gly Arg Val Arg Leu Met Val
   420 425 430
   Thr Gly Ala Ala Pro Val Ser Ala Thr Val Leu Thr Phe Leu Arg Ala
   435 440 445
   Ala Leu Gly Cys Gln Phe Tyr Glu Gly Tyr Gly Gln Thr Glu Cys Thr
   450 455 460
   Ala Gly Cys Cys Leu Thr Met Pro Gly Asp Trp Thr Ala Gly His Val
   465 470 475 480
   Gly Ala Pro Met Pro Cys Asn Leu Ile Lys Leu Val Asp Val Glu Glu
   485 490 495
   Met Asn Tyr Met Ala Ala Glu Gly Glu Gly Glu Val Cys Val Lys Gly
   500 505 510
   Pro Asn Val Phe Gln Gly Tyr Leu Lys Asp Pro Ala Lys Thr Ala Glu
   515 520 525
   Ala Leu Asp Lys Asp Gly Trp Leu His Thr Gly Asp Ile Gly Lys Trp
   530 535 540
   Leu Pro Asn Gly Thr Leu Lys Ile Ile Asp Arg Lys Lys His Ile Phe
   545 550 555 560
   Lys Leu Ala Gln Gly Glu Tyr Ile Ala Pro Glu Lys Ile Glu Asn Ile
   565 570 575
   Tyr Met Arg Ser Glu Pro Val Ala Gln Val Phe Val His Gly Glu Ser
   580 585 590
   Leu Gln Ala Phe Leu Ile Ala Ile Val Val Pro Asp Val Glu Thr Leu
   595 600 605
   Cys Ser Trp Ala Gln Lys Arg Gly Phe Glu Gly Ser Phe Glu Glu Leu
   610 615 620
   Cys Arg Asn Lys Asp Val Lys Lys Ala Ile Leu Glu Asp Met Val Arg
   625 630 635 640
   Leu Gly Lys Asp Ser Gly Leu Lys Pro Phe Glu Gln Val Lys Gly Ile
                   645                 650                 655
   Thr Leu His Pro Glu Leu Phe Ser Ile Asp Asn Gly Leu Leu Thr Pro
               660                 665                 670
   Thr Met Lys Ala Lys Arg Pro Glu Leu Arg Asn Tyr Phe Arg Ser Gln
           675                 680                 685
   Ile Asp Asp Leu Tyr Ser Thr Ile Lys Val
       690                 695
<210> 13
   <211> 552
   <212> PRT
   <213> Homo sapiens
<400> 13
   Met Leu Asp His Lys Asp Leu Glu Ala Glu Ile His Pro Leu Lys Asn
   1 5 10 15
   Glu Glu Arg Lys Ser Gln Glu Asn Leu Gly Asn Pro Ser Lys Asn Glu
   20 25 30
   Asp Asn Val Lys Ser Ala Pro Pro Gln Ser Arg Leu Ser Arg Cys Arg
   35 40 45
   Ala Ala Ala Phe Phe Leu Ser Leu Phe Leu Cys Leu Phe Val Val Phe
   50 55 60
   Val Val Ser Phe Val Ile Pro Cys Pro Asp Arg Pro Ala Ser Gln Arg
   65 70 75 80
   Met Trp Arg Ile Asp Tyr Ser Ala Ala Val Ile Tyr Asp Phe Leu Ala
   85 90 95
   Val Asp Asp Ile Asn Gly Asp Arg Ile Gln Asp Val Leu Phe Leu Tyr
   100 105 110
   Lys Asn Thr Asn Ser Ser Asn Asn Phe Ser Arg Ser Cys Val Asp Glu
   115 120 125
   Gly Phe Ser Ser Pro Cys Thr Phe Ala Ala Ala Val Ser Gly Ala Asn
   130 135 140
   Gly Ser Thr Leu Trp Glu Arg Pro Val Ala Gln Asp Val Ala Leu Val
   145 150 155 160
   Glu Cys Ala Val Pro Gln Pro Arg Gly Ser Glu Ala Pro Ser Ala Cys
   165 170 175
   Ile Leu Val Gly Arg Pro Ser Ser Phe Ile Ala Val Asn Leu Phe Thr
   180 185 190
   Gly Glu Thr Leu Trp Asn His Ser Ser Ser Phe Ser Gly Asn Ala Ser
   195 200 205
   Ile Leu Ser Pro Leu Leu Gln Val Pro Asp Val Asp Gly Asp Gly Ala
   210 215 220
   Pro Asp Leu Leu Val Leu Thr Gln Glu Arg Glu Glu Val Ser Gly His
   225 230 235 240
   Leu Tyr Ser Gly Ser Thr Gly His Gln Ile Gly Leu Arg Gly Ser Leu
   245 250 255
   Gly Val Asp Gly Glu Ser Gly Phe Leu Leu His Val Thr Arg Thr Gly
   260 265 270
   Ala His Tyr Ile Leu Phe Pro Cys Ala Ser Ser Leu Cys Gly Cys Ser
   275 280 285
   Val Lys Gly Leu Tyr Glu Lys Val Thr Gly Ser Gly Gly Pro Phe Lys
   290 295 300
   Ser Asp Pro His Trp Glu Ser Met Leu Asn Ala Thr Thr Arg Arg Met
   305 310 315 320
   Leu Ser His Ser Ser Gly Ala Val Arg Tyr Leu Met His Val Pro Gly
   325 330 335
   Asn Ala Gly Ala Asp Val Leu Leu Val Gly Ser Glu Ala Phe Val Leu
   340 345 350
   Leu Asp Gly Gln Glu Leu Thr Pro Arg Trp Thr Pro Lys Ala Ala His
   355 360 365
   Val Leu Arg Lys Pro Ile Phe Gly Arg Tyr Lys Pro Asp Thr Leu Ala
   370 375 380
   Val Ala Val Glu Asn Gly Thr Gly Thr Asp Arg Gln Ile Leu Phe Leu
   385 390 395 400
   Asp Leu Gly Thr Gly Ala Val Leu Cys Ser Leu Ala Leu Pro Ser Leu
   405 410 415
   Pro Gly Gly Pro Leu Ser Ala Ser Leu Pro Thr Ala Asp His Arg Ser
   420 425 430
   Ala Phe Phe Phe Trp Gly Leu His Glu Leu Gly Ser Thr Ser Glu Thr
   435 440 445
   Glu Thr Gly Glu Ala Arg His Ser Leu Tyr Met Phe His Pro Thr Leu
   450 455 460
   Pro Arg Val Leu Leu Glu Leu Ala Asn Val Ser Thr His Ile Val Ala
   465 470 475 480
   Phe Asp Ala Val Leu Phe Glu Pro Ser Arg His Ala Ala Tyr Ile Leu
   485 490 495
   Leu Thr Gly Pro Ala Asp Ser Glu Ala Pro Gly Leu Val Ser Val Ile
   500 505 510
   Lys His Lys Val Arg Asp Leu Val Pro Ser Ser Arg Val Val Arg Leu
   515 520 525
   Gly Glu Gly Gly Pro Asp Ser Asp Gln Ala Ile Arg Asp Arg Phe Ser
   530 535 540
   Arg Leu Arg Tyr Gln Ser Glu Ala
   545 550
<210> 14
   <211> 963
   <212> PRT
   <213> Homo sapiens
<400> 14
   Met Gly Leu Pro Glu Pro Gly Pro Leu Arg Leu Leu Ala Leu Leu Leu
   1               5                   10                  15
   Leu Leu Leu Leu Leu Leu Leu Leu Gln Leu Gln His Leu Ala Ala Ala
               20                  25                  30
   Ala Ala Asp Pro Leu Leu Gly Gly Gln Gly Pro Ala Lys Asp Cys Glu
           35                  40                  45
   Lys Asp Gln Phe Gln Cys Arg Asn Glu Arg Cys Ile Pro Ser Val Trp
       50                  55                  60
   Arg Cys Asp Glu Asp Asp Asp Cys Leu Asp His Ser Asp Glu Asp Asp
   65                  70                  75                  80
   Cys Pro Lys Lys Thr Cys Ala Asp Ser Asp Phe Thr Cys Asp Asn Gly
                   85                  90                  95
   His Cys Ile His Glu Arg Trp Lys Cys Asp Gly Glu Glu Glu Cys Pro
   100 105 110
   Asp Gly Ser Asp Glu Ser Glu Ala Thr Cys Thr Lys Gln Val Cys Pro
   115 120 125
   Ala Glu Lys Leu Ser Cys Gly Pro Thr Ser His Lys Cys Val Pro Ala
   130 135 140
   Ser Trp Arg Cys Asp Gly Glu Lys Asp Cys Glu Gly Gly Ala Asp Glu
   145 150 155 160
   Ala Gly Cys Ala Thr Leu Cys Ala Pro His Glu Phe Gln Cys Gly Asn
   165 170 175
   Arg Ser Cys Leu Ala Ala Val Phe Val Cys Asp Gly Asp Asp Asp Cys
   180 185 190
   Gly Asp Gly Ser Asp Glu Arg Gly Cys Ala Asp Pro Ala Cys Gly Pro
   195 200 205
   Arg Glu Phe Arg Cys Gly Gly Asp Gly Gly Gly Ala Cys Ile Pro Glu
   210 215 220
   Arg Trp Val Cys Asp Arg Gln Phe Asp Cys Glu Asp Arg Ser Asp Glu
   225 230 235 240
   Ala Ala Glu Leu Cys Gly Arg Pro Gly Pro Gly Ala Thr Ser Ala Pro
   245 250 255
   Ala Ala Cys Ala Thr Ala Ser Gln Phe Ala Cys Arg Ser Gly Glu Cys
   260 265 270
   Val His Leu Gly Trp Arg Cys Asp Gly Asp Arg Asp Cys Lys Asp Lys
   275 280 285
   Ser Asp Glu Ala Asp Cys Pro Leu Gly Thr Cys Arg Gly Asp Glu Phe
   290 295 300
   Gln Cys Gly Asp Gly Thr Cys Val Leu Ala Ile Lys His Cys Asn Gln
   305 310 315 320
   Glu Gln Asp Cys Pro Asp Gly Ser Asp Glu Ala Gly Cys Leu Gln Gly
   325 330 335
   Leu Asn Glu Cys Leu His Asn Asn Gly Gly Cys Ser His Ile Cys Thr
   340 345 350
   Asp Leu Lys Ile Gly Phe Glu Cys Thr Cys Pro Ala Gly Phe Gln Leu
   355 360 365
   Leu Asp Gln Lys Thr Cys Gly Asp Ile Asp Glu Cys Lys Asp Pro Asp
   370 375 380
   Ala Cys Ser Gln Ile Cys Val Asn Tyr Lys Gly Tyr Phe Lys Cys Glu
   385 390 395 400
   Cys Tyr Pro Gly Tyr Glu Met Asp Leu Leu Thr Lys Asn Cys Lys Ala
   405 410 415
   Ala Ala Gly Lys Ser Pro Ser Leu Ile Phe Thr Asn Arg His Glu Val
   420 425 430
   Arg Arg Ile Asp Leu Val Lys Arg Asn Tyr Ser Arg Leu Ile Pro Met
   435 440 445
   Leu Lys Asn Val Val Ala Leu Asp Val Glu Val Ala Thr Asn Arg Ile
   450 455 460
   Tyr Trp Cys Asp Leu Ser Tyr Arg Lys Ile Tyr Ser Ala Tyr Met Asp
   465 470 475 480
   Lys Ala Ser Asp Pro Lys Glu Gln Glu Val Leu Ile Asp Glu Gln Leu
   485 490 495
   His Ser Pro Glu Gly Leu Ala Val Asp Trp Val His Lys His Ile Tyr
   500 505 510
   Trp Thr Asp Ser Gly Asn Lys Thr Ile Ser Val Ala Thr Val Asp Gly
   515 520 525
   Gly Arg Arg Arg Thr Leu Phe Ser Arg Asn Leu Ser Glu Pro Arg Ala
   530 535 540
   Ile Ala Val Asp Pro Leu Arg Gly Phe Met Tyr Trp Ser Asp Trp Gly
   545 550 555 560
   Asp Gln Ala Lys Ile Glu Lys Ser Gly Leu Asn Gly Val Asp Arg Gln
   565 570 575
   Thr Leu Val Ser Asp Asn Ile Glu Trp Pro Asn Gly Ile Thr Leu Asp
   580 585 590
   Leu Leu Ser Gln Arg Leu Tyr Trp Val Asp Ser Lys Leu His Gln Leu
   595 600 605
   Ser Ser Ile Asp Phe Ser Gly Gly Asn Arg Lys Thr Leu Ile Ser Ser
   610 615 620
   Thr Asp Phe Leu Ser His Pro Phe Gly Ile Ala Val Phe Glu Asp Lys
   625 630 635 640
   Val Phe Trp Thr Asp Leu Glu Asn Glu Ala Ile Phe Ser Ala Asn Arg
   645 650 655
   Leu Asn Gly Leu Glu Ile Ser Ile Leu Ala Glu Asn Leu Asn Asn Pro
   660 665 670
   His Asp Ile Val Ile Phe His Glu Leu Lys Gln Pro Arg Ala Pro Asp
   675 680 685
   Ala Cys Glu Leu Ser Val Gln Pro Asn Gly Gly Cys Glu Tyr Leu Cys
   690 695 700
   Leu Pro Ala Pro Gln Ile Ser Ser His Ser Pro Lys Tyr Thr Cys Ala
   705 710 715 720
   Cys Pro Asp Thr Met Trp Leu Gly Pro Asp Met Lys Arg Cys Tyr Arg
   725 730 735
   Ala Pro Gln Ser Thr Ser Thr Thr Thr Leu Ala Ser Thr Met Thr Arg
   740 745 750
   Thr Val Pro Ala Thr Thr Arg Ala Pro Gly Thr Thr Val His Arg Ser
   755 760 765
   Thr Tyr Gln Asn His Ser Thr Glu Thr Pro Ser Leu Thr Ala Ala Val
   770 775 780
   Pro Ser Ser Val Ser Val Pro Arg Ala Pro Ser Ile Ser Pro Ser Thr
   785 790 795 800
   Leu Ser Pro Ala Thr Ser Asn His Ser Gln His Tyr Ala Asn Glu Asp
   805 810 815
   Ser Lys Met Gly Ser Thr Val Thr Ala Ala Val Ile Gly Ile Ile Val
   820 825 830
   Pro Ile Val Val Ile Ala Leu Leu Cys Met Ser Gly Tyr Leu Ile Trp
   835 840 845
   Arg Asn Trp Lys Arg Lys Asn Thr Lys Ser Met Asn Phe Asp Asn Pro
   850 855 860
   Val Tyr Arg Lys Thr Thr Glu Glu Glu Asp Glu Asp Glu Leu His Ile
   865 870 875 880
   Gly Arg Thr Ala Gln Ile Gly His Val Tyr Pro Ala Ala Ile Ser Ser
   885 890 895
   Phe Asp Arg Pro Leu Trp Ala Glu Pro Cys Leu Gly Glu Thr Arg Glu
   900 905 910
   Pro Glu Asp Pro Ala Pro Ala Leu Lys Glu Leu Phe Val Leu Pro Gly
   915 920 925
   Glu Pro Arg Ser Gln Leu His Gln Leu Pro Lys Asn Pro Leu Ser Glu
   930 935 940
   Leu Pro Val Val Lys Ser Lys Arg Val Ala Leu Ser Leu Glu Asp Asp
   945 950 955 960
   Gly Leu Pro
<210> 15
   <211> 150
   <212> PRT
   <213> Homo sapiens
<400> 15
   Met Ser Gly Ser Met Ala Thr Ala Glu Ala Ser Gly Ser Asp Gly Lys
   1               5                   10                  15
   Gly Gln Glu Val Glu Thr Ser Val Thr Tyr Tyr Arg Leu Glu Glu Val
               20                  25                  30
   Ala Lys Arg Asn Ser Leu Lys Glu Leu Trp Leu Val Ile His Gly Arg
           35                  40                  45
   Val Tyr Asp Val Thr Arg Phe Leu Asn Glu His Pro Gly Gly Glu Glu
       50                  55                  60
   Val Leu Leu Glu Gln Ala Gly Val Asp Ala Ser Glu Ser Phe Glu Asp
   65                  70                  75                  80
   Val Gly His Ser Ser Asp Ala Arg Glu Met Leu Lys Gln Tyr Tyr Ile
                   85                  90                  95
   Gly Asp Ile His Pro Ser Asp Leu Lys Pro Glu Ser Gly Ser Lys Asp
               100                 105                 110
   Pro Ser Lys Asn Asp Thr Cys Lys Ser Cys Trp Ala Tyr Trp Ile Leu
           115                 120                 125
   Pro Ile Ile Gly Ala Val Leu Leu Gly Phe Leu Tyr Arg Tyr Tyr Thr
           130                 135                 140
   Ser Glu Ser Lys Ser Ser
   145                 150
<210> 16
   <211> 327
   <212> PRT
   <213> Homo sapiens
<400> 16
   Met Ala Ala Ala Ala Ala Ala Ala Ala Ala Thr Asn Gly Thr Gly Gly
   1 5 10 15
   Ser Ser Gly Met Glu Val Asp Ala Ala Val Val Pro Ser Val Met Ala
   20 25 30
   Cys Gly Val Thr Gly Ser Val Ser Val Ala Leu His Pro Leu Val Ile
   35 40 45
   Leu Asn Ile Ser Asp His Trp Ile Arg Met Arg Ser Gln Glu Gly Arg
   50 55 60
   Pro Val Gln Val Ile Gly Ala Leu Ile Gly Lys Gln Glu Gly Arg Asn
   65 70 75 80
   Ile Glu Val Met Asn Ser Phe Glu Leu Leu Ser His Thr Val Glu Glu
   85 90 95
   Lys Ile Ile Ile Asp Lys Glu Tyr Tyr Tyr Thr Lys Glu Glu Gln Phe
   100 105 110
   Lys Gln Val Phe Lys Glu Leu Glu Phe Leu Gly Trp Tyr Thr Thr Gly
   115 120 125
   Gly Pro Pro Asp Pro Ser Asp Ile His Val His Lys Gln Val Cys Glu
   130 135 140
   Ile Ile Glu Ser Pro Leu Phe Leu Lys Leu Asn Pro Met Thr Lys His
   145 150 155 160
   Thr Asp Leu Pro Val Ser Val Phe Glu Ser Val Ile Asp Ile Ile Asn
   165 170 175
   Gly Glu Ala Thr Met Leu Phe Ala Glu Leu Thr Tyr Thr Leu Ala Thr
   180 185 190
   Glu Glu Ala Glu Arg Ile Gly Val Asp His Val Ala Arg Met Thr Ala
   195 200 205
   Thr Gly Ser Gly Glu Asn Ser Thr Val Ala Glu His Leu Ile Ala Gln
       210                 215                 220
   His Ser Ala Ile Lys Met Leu His Ser Arg Val Lys Leu Ile Leu Glu
   225                 230                 235                 240
   Tyr Val Lys Ala Ser Glu Ala Gly Glu Val Pro Phe Asn His Glu Ile
                   245                 250                 255
   Leu Arg Glu Ala Tyr Ala Leu Cys His Cys Leu Pro Val Leu Ser Thr
               260                 265                 270
   Asp Lys Phe Lys Thr Asp Phe Tyr Asp Gln Cys Asn Asp Val Gly Leu
           275                 280                 285
   Met Ala Tyr Leu Gly Thr Ile Thr Lys Thr Cys Asn Thr Met Asn Gln
       290                 295                 300
   Phe Val Asn Lys Phe Asn Val Leu Tyr Asp Arg Gln Gly Ile Gly Arg
   305                 310                 315                 320
   Arg Met Arg Gly Leu Phe Phe
                   325
<210> 17
   <211> 343
   <212> PRT
   <213> Homo sapiens
<400> 17
   Met Ala Gln Lys Gly Val Leu Gly Pro Gly Gln Leu Gly Ala Val Ala
   1               5                   10                  15
   Ile Leu Leu Tyr Leu Gly Leu Leu Arg Ser Gly Thr Gly Lys Glu Gly
               20                  25                  30
   Ala Ala Ala Pro Cys Gly Val Ala Pro Gln Ala Arg Ile Thr Gly Gly
           35                  40                  45
   Ser Ser Ala Val Ala Gly Gln Trp Pro Trp Gln Val Ser Ile Thr Tyr
       50                  55                  60
   Glu Gly Val His Val Cys Gly Gly Ser Leu Val Ser Glu Gln Trp Val
   65                  70                  75                  80
   Leu Ser Ala Ala His Cys Phe Pro Ser Glu His His Lys Glu Ala Tyr
                   85                  90                  95
   Glu Val Lys Leu Gly Ala His Gln Leu Asp Ser Tyr Ser Glu Asp Ala
               100                 105                 110
   Lys Val Ser Thr Leu Lys Asp Ile Ile Pro His Pro Ser Tyr Leu Gln
           115                 120                 125
   Glu Gly Ser Gln Gly Asp Ile Ala Leu Leu Gln Leu Ser Arg Pro Ile
       130                 135                 140
   Thr Phe Ser Arg Tyr Ile Arg Pro Ile Cys Leu Pro Ala Ala Asn Ala
   145                 150                 155                 160
   Ser Phe Pro Asn Gly Leu His Cys Thr Val Thr Gly Trp Gly His Val
                   165                 170                 175
   Ala Pro Ser Val Ser Leu Leu Thr Pro Lys Pro Leu Gln Gln Leu Glu
               180                 185                 190
   Val Pro Leu Ile Ser Arg Glu Thr Cys Asn Cys Leu Tyr Asn Ile Asp
           195                 200                 205
   Ala Lys Pro Glu Glu Pro His Phe Val Gln Glu Asp Met Val Cys Ala
       210                 215                 220
   Gly Tyr Val Glu Gly Gly Lys Asp Ala Cys Gln Gly Asp Ser Gly Gly
   225                 230                 235                 240
   Pro Leu Ser Cys Pro Val Glu Gly Leu Trp Tyr Leu Thr Gly Ile Val
                   245                 250                 255
   Ser Trp Gly Asp Ala Cys Gly Ala Arg Asn Arg Pro Gly Val Tyr Thr
               260                 265                 270
   Leu Ala Ser Ser Tyr Ala Ser Trp Ile Gln Ser Lys Val Thr Glu Leu
           275                 280                 285
   Gln Pro Arg Val Val Pro Gln Thr Gln Glu Ser Gln Pro Asp Ser Asn
       290                 295                 300
   Leu Cys Gly Ser His Leu Ala Phe Ser Ser Ala Pro Ala Gln Gly Leu
   305                 310                 315                 320
   Leu Arg Pro Ile Leu Phe Leu Pro Leu Gly Leu Ala Leu Gly Leu Leu
                   325                 330                 335
   Ser Pro Trp Leu Ser Glu His
               340
<210> 18
   <211> 198
   <212> PRT
   <213> Homo sapiens
<400> 18
   Met Ala Pro Ala Arg Leu Phe Ala Leu Leu Leu Phe Phe Val Gly Gly
   1               5                   10                  15
   Val Ala Glu Ser Ile Arg Glu Thr Glu Val Ile Asp Pro Gln Asp Leu
               20                  25                  30
   Leu Glu Gly Arg Tyr Phe Ser Gly Ala Leu Pro Asp Asp Glu Asp Val
           35                  40                  45
   Val Gly Pro Gly Gln Glu Ser Asp Asp Phe Glu Leu Ser Gly Ser Gly
       50                  55                  60
   Asp Leu Asp Asp Leu Glu Asp Ser Met Ile Gly Pro Glu Val Val His
   65                  70                  75                  80
   Pro Leu Val Pro Leu Asp Asn His Ile Pro Glu Arg Ala Gly Ser Gly
                   85                  90                  95
   Ser Gln Val Pro Thr Glu Pro Lys Lys Leu Glu Glu Asn Glu Val Ile
               100                 105                 110
   Pro Lys Arg Ile Ser Pro Val Glu Glu Ser Glu Asp Val Ser Asn Lys
           115                 120                 125
   Val Ser Met Ser Ser Thr Val Gln Gly Ser Asn Ile Phe Glu Arg Thr
       130                 135                 140
   Glu Val Leu Ala Ala Leu Ile Val Gly Gly Ile Val Gly Ile Leu Phe
   145                 150                 155                 160
   Ala Val Phe Leu Ile Leu Leu Leu Met Tyr Arg Met Lys Lys Lys Asp
                   165                 170                 175
   Glu Gly Ser Tyr Asp Leu Gly Lys Lys Pro Ile Tyr Lys Lys Ala Pro
               180                 185                 190
   Thr Asn Glu Phe Tyr Ala
           195
<210> 19
   <211> 492
   <212> PRT
   <213> Homo sapiens
<400> 19
<210> 20
   <211> 180
   <212> PRT
   <213> Homo sapiens
<400> 20
   Met Ala Ser Thr Ser Tyr Asp Tyr Cys Arg Val Pro Met Glu Asp Gly
   1               5                   10                  15
   Asp Lys Arg Cys Lys Leu Leu Leu Gly Ile Gly Ile Leu Val Leu Leu
               20                  25                  30
   Ile Ile Val Ile Leu Gly Val Pro Leu Ile Ile Phe Thr Ile Lys Ala
           35                  40                  45
   Asn Ser Glu Ala Cys Arg Asp Gly Leu Arg Ala Val Met Glu Cys Arg
       50                  55                  60
   Asn Val Thr His Leu Leu Gln Gln Glu Leu Thr Glu Ala Gln Lys Gly
   65                  70                  75                  80
   Phe Gln Asp Val Glu Ala Gln Ala Ala Thr Cys Asn His Thr Val Met
                   85                  90                  95
   Ala Leu Met Ala Ser Leu Asp Ala Glu Lys Ala Gln Gly Gln Lys Lys
               100                 105                 110
   Val Glu Glu Leu Glu Gly Glu Ile Thr Thr Leu Asn His Lys Leu Gln
           115                 120                 125
   Asp Ala Ser Ala Glu Val Glu Arg Leu Arg Arg Glu Asn Gln Val Leu
       130                 135                 140
   Ser Val Arg Ile Ala Asp Lys Lys Tyr Tyr Pro Ser Ser Gln Asp Ser
   145                 150                 155                 160
   Ser Ser Ala Ala Ala Pro Gln Leu Leu Ile Val Leu Leu Gly Leu Ser
                   165                 170                 175
   Ala Leu Leu Gln
               180
<210> 21
   <211> 341
   <212> PRT
   <213> Homo sapiens
<400> 21
   Met Arg Lys Val Val Leu Ile Thr Gly Ala Ser Ser Gly Ile Gly Leu
   1               5                   10                  15
   Ala Leu Cys Lys Arg Leu Leu Ala Glu Asp Asp Glu Leu His Leu Cys
               20                  25                  30
   Leu Ala Cys Arg Asn Met Ser Lys Ala Glu Ala Val Cys Ala Ala Leu
           35                  40                  45
   Leu Ala Ser His Pro Thr Ala Glu Val Thr Ile Val Gln Val Asp Val
   50 55 60
   Ser Asn Leu Gln Ser Val Phe Arg Ala Ser Lys Glu Leu Lys Gln Arg
   65 70 75 80
   Phe Gln Arg Leu Asp Cys Ile Tyr Leu Asn Ala Gly Ile Met Pro Asn
   85 90 95
   Pro Gln Leu Asn Ile Lys Ala Leu Phe Phe Gly Leu Phe Ser Arg Lys
   100 105 110
   Val Ile His Met Phe Ser Thr Ala Glu Gly Leu Leu Thr Gln Gly Asp
   115 120 125
   Lys Ile Thr Ala Asp Gly Leu Gln Glu Val Phe Glu Thr Asn Val Phe
   130 135 140
   Gly His Phe Ile Leu Ile Arg Glu Leu Glu Pro Leu Leu Cys His Ser
   145 150 155 160
   Asp Asn Pro Ser Gln Leu Ile Trp Thr Ser Ser Arg Ser Ala Arg Lys
   165 170 175
   Ser Asn Phe Ser Leu Glu Asp Phe Gln His Ser Lys Gly Lys Glu Pro
   180 185 190
   Tyr Ser Ser Ser Lys Tyr Ala Thr Asp Leu Leu Ser Val Ala Leu Asn
   195 200 205
   Arg Asn Phe Asn Gln Gln Gly Leu Tyr Ser Asn Val Ala Cys Pro Gly
   210 215 220
   Thr Ala Leu Thr Asn Leu Thr Tyr Gly Ile Leu Pro Pro Phe Ile Trp
   225 230 235 240
   Thr Leu Leu Met Pro Ala Ile Leu Leu Leu Arg Phe Phe Ala Asn Ala
   245 250 255
   Phe Thr Leu Thr Pro Tyr Asn Gly Thr Glu Ala Leu Val Trp Leu Phe
   260 265 270
   His Gln Lys Pro Glu Ser Leu Asn Pro Leu Ile Lys Tyr Leu Ser Ala
   275 280 285
   Thr Thr Gly Phe Gly Arg Asn Tyr Ile Met Thr Gln Lys Met Asp Leu
   290 295 300
   Asp Glu Asp Thr Ala Glu Lys Phe Tyr Gln Lys Leu Leu Glu Leu Glu
   305 310 315 320
   Lys His Ile Arg Val Thr Ile Gln Lys Thr Asp Asn Gln Ala Arg Leu
   325 330 335
   Ser Gly Ser Cys Leu
   340
<210> 22
   <211> 454
   <212> PRT
   <213> Homo sapiens
<400> 22
<210> 23
   <211> 528
   <212> PRT
   <213> Homo sapiens
<400> 23
   Met Arg Cys Ala Leu Ala Leu Ser Ala Leu Leu Leu Leu Leu Ser Thr
   1 5 10 15
   Pro Pro Leu Leu Pro Ser Ser Pro Ser Pro Ser Pro Ser Pro Ser Gln
   20 25 30
   Asn Glu Thr Ala Thr Gln Thr Thr Thr Asp Ser Ser Asn Lys Thr Ala
   35 40 45
   Pro Thr Pro Ala Ser Ser Val Thr Ile Met Ala Thr Asp Thr Ala Gln
   50 55 60
   Gln Ser Thr Val Pro Thr Ser Lys Ala Asn Glu Ile Leu Ala Ser Val
   65 70 75 80
   Lys Ala Thr Thr Leu Gly Val Ser Ser Asp Ser Pro Gly Thr Thr Thr
   85 90 95
   Leu Ala Gln Gln Val Ser Gly Pro Val Asn Thr Thr Val Ala Arg Gly
   100 105 110
   Gly Gly Ser Gly Asn Pro Thr Thr Thr Ile Glu Ser Pro Lys Ser Thr
   115 120 125
   Lys Ser Ala Asp Thr Thr Thr Val Ala Thr Ser Thr Ala Thr Ala Lys
   130 135 140
   Pro Asn Thr Thr Ser Ser Gln Asn Gly Ala Glu Asp Thr Thr Asn Ser
   145 150 155 160
   Gly Gly Lys Ser Ser His Ser Val Thr Thr Asp Leu Thr Ser Thr Lys
   165 170 175
   Ala Glu His Leu Thr Thr Pro His Pro Thr Ser Pro Leu Ser Pro Arg
   180 185 190
   Gln Pro Thr Ser Thr His Pro Val Ala Thr Pro Thr Ser Ser Gly His
   195 200 205
   Asp His Leu Met Lys Ile Ser Ser Ser Ser Ser Thr Val Ala Ile Pro
       210                 215                 220
   Gly Tyr Thr Phe Thr Ser Pro Gly Met Thr Thr Thr Leu Pro Ser Ser
   225                 230                 235                 240
   Val Ile Ser Gln Arg Thr Gln Gln Thr Ser Ser Gln Met Pro Ala Ser
                   245                 250                 255
   Ser Thr Ala Pro Ser Ser Gln Glu Thr Val Gln Pro Thr Ser Pro Ala
               260                 265                 270
   Thr Ala Leu Arg Thr Pro Thr Leu Pro Glu Thr Met Ser Ser Ser Pro
           275                 280                 285
   Thr Ala Ala Ser Thr Thr His Arg Tyr Pro Lys Thr Pro Ser Pro Thr
       290                 295                 300
   Val Ala His Glu Ser Asn Trp Val Thr Pro Ala Gly Val Gly Thr Gln
   305                 310                 315                 320
   Thr Arg Val Glu Glu Ala Leu Arg Gln Ala Leu Thr His Ser Leu Leu
                   325                 330                 335
   Pro Ala Gly Gly Ala Ser Asp Glu Lys Leu Ile Ser Leu Ile Cys Arg
               340                 345                 350
   Ala Val Lys Ala Thr Phe Asn Pro Ala Gln Asp Lys Cys Gly Ile Arg
           355                 360                 365
   Leu Ala Ser Val Pro Gly Ser Gln Thr Val Val Val Lys Glu Ile Thr
       370                 375                 380
   Ile His Thr Lys Leu Pro Ala Lys Asp Val Tyr Glu Arg Leu Lys Asp
   385                 390                 395                 400
   Lys Trp Asp Glu Leu Lys Glu Ala Gly Val Ser Asp Met Lys Leu Gly
                   405                 410                 415
   Asp Gln Gly Pro Pro Glu Glu Ala Glu Asp Arg Phe Ser Met Pro Leu
               420                 425                 430
   Ile Ile Thr Ile Val Cys Met Ala Ser Phe Leu Leu Leu Val Ala Ala
           435                 440                 445
   Leu Tyr Gly Cys Cys His Gln Arg Leu Ser Gln Arg Lys Asp Gln Gln
       450                 455                 460
   Arg Leu Thr Glu Glu Leu Gln Thr Val Glu Asn Gly Tyr His Asp Asn
   465 470 475 480
   Pro Thr Leu Glu Val Met Glu Thr Ser Ser Glu Met Gln Glu Lys Lys
   485 490 495
   Val Val Ser Leu Asn Gly Glu Leu Gly Asp Ser Trp Ile Val Pro Leu
   500 505 510
   Asp Asn Leu Thr Lys Asp Asp Leu Asp Glu Glu Glu Asp Thr His Leu
   515 520 525
<210> 24
   <211> 475
   <212> PRT
   <213> Homo sapiens
<400> 24
<210> 25
   <211> 1241
   <212> PRT
   <213> Homo sapiens
<400> 25
   Met Ser Ser Ala Pro Arg Arg Pro Ala Lys Gly Ala Asp Ser Phe Cys
   1               5                   10                  15
   Thr Pro Glu Pro Glu Ser Leu Gly Pro Gly Thr Pro Gly Phe Pro Glu
               20                  25                  30
   Gln Glu Glu Asp Glu Leu His Arg Thr Leu Gly Val Glu Arg Phe Glu
           35                  40                  45
   Glu Ile Leu Gln Glu Ala Gly Ser Arg Gly Gly Glu Glu Pro Gly Arg
       50                  55                  60
   Ser Tyr Gly Glu Glu Asp Phe Glu Tyr His Arg Gln Ser Ser His His
   65                  70                  75                  80
   Ile His His Pro Leu Ser Thr His Leu Pro Pro Asp Ala Arg Arg Arg
                   85                  90                  95
   Lys Thr Pro Gln Gly Pro Gly Arg Lys Pro Arg Arg Arg Pro Gly Ala
               100                 105                 110
   Ser Pro Thr Gly Glu Thr Pro Thr Ile Glu Glu Gly Glu Glu Asp Glu
           115                 120                 125
   Asp Glu Ala Ser Glu Ala Glu Gly Ala Arg Ala Leu Thr Gln Pro Ser
       130                 135                 140
   Pro Val Ser Thr Pro Ser Ser Val Gln Phe Phe Leu Gln Glu Asp Asp
   145                 150                 155                 160
   Ser Ala Asp Arg Lys Ala Glu Arg Thr Ser Pro Ser Ser Pro Ala Pro
                   165                 170                 175
   Leu Pro His Gln Glu Ala Thr Pro Arg Ala Ser Lys Gly Ala Gln Ala
               180                 185                 190
   Gly Thr Gln Val Glu Glu Ala Glu Ala Glu Ala Val Ala Val Ala Ser
           195                 200                 205
   Gly Thr Ala Gly Gly Asp Asp Gly Gly Ala Ser Gly Arg Pro Leu Pro
       210                 215                 220
   Lys Ala Gln Pro Gly His Arg Ser Tyr Asn Leu Gln Glu Arg Arg Arg
   225                 230                 235                 240
   Ile Gly Ser Met Thr Gly Ala Glu Gln Ala Leu Leu Pro Arg Val Pro
                   245                 250                 255
   Thr Asp Glu Ile Glu Ala Gln Thr Leu Ala Thr Ala Asp Leu Asp Leu
               260                 265                 270
   Met Lys Ser His Arg Phe Glu Asp Val Pro Gly Val Arg Arg His Leu
           275                 280                 285
   Val Arg Lys Asn Ala Lys Gly Ser Thr Gln Ser Gly Arg Glu Gly Arg
       290                 295                 300
   Glu Pro Gly Pro Thr Pro Arg Ala Arg Pro Arg Ala Pro His Lys Pro
   305                 310                 315                 320
   His Glu Val Phe Val Glu Leu Asn Glu Leu Leu Leu Asp Lys Asn Gln
                   325                 330                 335
   Glu Pro Gln Trp Arg Glu Thr Ala Arg Trp Ile Lys Phe Glu Glu Asp
               340                 345                 350
   Val Glu Glu Glu Thr Glu Arg Trp Gly Lys Pro His Val Ala Ser Leu
           355                 360                 365
   Ser Phe Arg Ser Leu Leu Glu Leu Arg Arg Thr Leu Ala His Gly Ala
       370                 375                 380
   Val Leu Leu Asp Leu Asp Gln Gln Thr Leu Pro Gly Val Ala His Gln
   385                 390                 395                 400
   Val Val Glu Gln Met Val Ile Ser Asp Gln Ile Lys Ala Glu Asp Arg
                   405                 410                 415
   Ala Asn Val Leu Arg Ala Leu Leu Leu Lys His Ser His Pro Ser Asp
               420                 425                 430
   Glu Lys Asp Phe Ser Phe Pro Arg Asn Ile Ser Ala Gly Ser Leu Gly
           435                 440                 445
   Ser Leu Leu Gly His His His Gly Gln Gly Ala Glu Ser Asp Pro His
   450 455 460
   Val Thr Glu Pro Leu Met Gly Gly Val Pro Glu Thr Arg Leu Glu Val
   465 470 475 480
   Glu Arg Glu Arg Glu Leu Pro Pro Pro Ala Pro Pro Ala Gly Ile Thr
   485 490 495
   Arg Ser Lys Ser Lys His Glu Leu Lys Leu Leu Glu Lys Ile Pro Glu
   500 505 510
   Asn Ala Glu Ala Thr Val Val Leu Val Gly Cys Val Glu Phe Leu Ser
   515 520 525
   Arg Pro Thr Met Ala Phe Val Arg Leu Arg Glu Ala Val Glu Leu Asp
   530 535 540
   Ala Val Leu Glu Val Pro Val Pro Val Arg Phe Leu Phe Leu Leu Leu
   545 550 555 560
   Gly Pro Ser Ser Ala Asn Met Asp Tyr His Glu Ile Gly Arg Ser Ile
   565 570 575
   Ser Thr Leu Met Ser Asp Lys Gln Phe His Glu Ala Ala Tyr Leu Ala
   580 585 590
   Asp Glu Arg Glu Asp Leu Leu Thr Ala Ile Asn Ala Phe Leu Asp Cys
   595 600 605
   Ser Val Val Leu Pro Pro Ser Glu Val Gln Gly Glu Glu Leu Leu Arg
   610 615 620
   Ser Val Ala His Phe Gln Arg Gln Met Leu Lys Lys Arg Glu Glu Gln
   625 630 635 640
   Gly Arg Leu Leu Pro Thr Gly Ala Gly Leu Glu Pro Lys Ser Ala Gln
   645 650 655
   Asp Lys Ala Leu Leu Gln Met Val Glu Ala Ala Gly Ala Ala Glu Asp
   660 665 670
   Asp Pro Leu Arg Arg Thr Gly Arg Pro Phe Gly Gly Leu Ile Arg Asp
   675 680 685
   Val Arg Arg Arg Tyr Pro His Tyr Leu Ser Asp Phe Arg Asp Ala Leu
   690 695 700
   Asp Pro Gln Cys Leu Ala Ala Val Ile Phe Ile Tyr Phe Ala Ala Leu
   705 710 715 720
   Ser Pro Ala Ile Thr Phe Gly Gly Leu Leu Gly Glu Lys Thr Gln Asp
   725 730 735
   Leu Ile Gly Val Ser Glu Leu Ile Met Ser Thr Ala Leu Gln Gly Val
   740 745 750
   Val Phe Cys Leu Leu Gly Ala Gln Pro Leu Leu Val Ile Gly Phe Ser
   755 760 765
   Gly Pro Leu Leu Val Phe Glu Glu Ala Phe Phe Ser Phe Cys Ser Ser
   770 775 780
   Asn His Leu Glu Tyr Leu Val Gly Arg Val Trp Ile Gly Phe Trp Leu
   785 790 795 800
   Val Phe Leu Ala Leu Leu Met Val Ala Leu Glu Gly Ser Phe Leu Val
   805 810 815
   Arg Phe Val Ser Arg Phe Thr Gln Glu Ile Phe Ala Phe Leu Ile Ser
   820 825 830
   Leu Ile Phe Ile Tyr Glu Thr Phe Tyr Lys Leu Val Lys Ile Phe Gln
   835 840 845
   Glu His Pro Leu His Gly Cys Ser Ala Ser Asn Ser Ser Glu Val Asp
   850 855 860
   Gly Gly Glu Asn Met Thr Trp Ala Gly Ala Arg Pro Thr Leu Gly Pro
   865 870 875 880
   Gly Asn Arg Ser Leu Ala Gly Gln Ser Gly Gln Gly Lys Pro Arg Gly
   885 890 895
   Gln Pro Asn Thr Ala Leu Leu Ser Leu Val Leu Met Ala Gly Thr Phe
   900 905 910
   Phe Ile Ala Phe Phe Leu Arg Lys Phe Lys Asn Ser Arg Phe Phe Pro
   915 920 925
   Gly Arg Ile Arg Arg Val Ile Gly Asp Phe Gly Val Pro Ile Ala Ile
   930 935 940
   Leu Ile Met Val Leu Val Asp Tyr Ser Ile Glu Asp Thr Tyr Thr Gln
   945 950 955 960
   Lys Leu Ser Val Pro Ser Gly Phe Ser Val Thr Ala Pro Glu Lys Arg
   965 970 975
   Gly Trp Val Ile Asn Pro Leu Gly Glu Lys Ser Pro Phe Pro Val Trp
   980 985 990
   Met Met Val Ala Ser Leu Leu Pro Ala Ile Leu Val Phe Ile Leu Ile
   995 1000 1005
   Phe Met Glu Thr Gln Ile Thr Thr Leu Ile Ile Ser Lys Lys Glu
   1010 1015 1020
   Arg Met Leu Gln Lys Gly Ser Gly Phe His Leu Asp Leu Leu Leu
   1025 1030 1035
   Ile Val Ala Met Gly Gly Ile Cys Ala Leu Phe Gly Leu Pro Trp
   1040 1045 1050
   Leu Ala Ala Ala Thr Val Arg Ser Val Thr His Ala Asn Ala Leu
   1055 1060 1065
   Thr Val Met Ser Lys Ala Val Ala Pro Gly Asp Lys Pro Lys Ile
   1070 1075 1080
   Gln Glu Val Lys Glu Gln Arg Val Thr Gly Leu Leu Val Ala Leu
   1085 1090 1095
   Leu Val Gly Leu Ser Ile Val Ile Gly Asp Leu Leu Arg Gln Ile
   1100 1105 1110
   Pro Leu Ala Val Leu Phe Gly Ile Phe Leu Tyr Met Gly Val Thr
   1115 1120 1125
   Ser Leu Asn Gly Ile Gln Phe Tyr Glu Arg Leu His Leu Leu Leu
   1130 1135 1140
   Met Pro Pro Lys His His Pro Asp Val Thr Tyr Val Lys Lys Val
   1145 1150 1155
   Arg Thr Leu Arg Met His Leu Phe Thr Ala Leu Gln Leu Leu Cys
   1160 1165 1170
   Leu Ala Leu Leu Trp Ala Val Met Ser Thr Ala Ala Ser Leu Ala
   1175 1180 1185
   Phe Pro Phe Ile Leu Ile Leu Thr Val Pro Leu Arg Met Val Val
   1190 1195 1200
   Leu Thr Arg Ile Phe Thr Asp Arg Glu Met Lys Cys Leu Asp Ala
   1205 1210 1215
   Asn Glu Ala Glu Pro Val Phe Asp Glu Arg Glu Gly Val Asp Glu
   1220 1225 1230
   Tyr Asn Glu Met Pro Met Pro Val
   1235 1240
<210> 26
   <211> 707
   <212> PRT
   <213> Homo sapiens
<400> 26
   Met Ala Ala Ala Val Ala Ala Ala Gly Arg Leu Gly Trp Leu Phe Ala
   1 5 10 15
   Ala Leu Cys Leu Gly Asn Ala Ala Gly Glu Ala Ala Pro Gly Pro Arg
   20 25 30
   Val Leu Gly Phe Cys Leu Glu Glu Asp Gly Ala Ala Gly Ala Gly Trp
   35 40 45
   Val Arg Gly Gly Ala Ala Arg Asp Thr Pro Asp Ala Thr Phe Leu Leu
   50 55 60
   Arg Leu Phe Gly Pro Gly Phe Ala Asn Ser Ser Trp Ser Trp Val Ala
   65 70 75 80
   Pro Glu Gly Ala Gly Cys Arg Glu Glu Ala Ala Ser Pro Ala Gly Glu
   85 90 95
   Trp Arg Ala Leu Leu Arg Leu Arg Leu Arg Ala Glu Ala Val Arg Pro
   100 105 110
   His Ser Ala Leu Leu Ala Val Arg Val Glu Pro Gly Gly Gly Ala Ala
   115 120 125
   Glu Glu Ala Ala Pro Pro Trp Ala Leu Gly Leu Gly Ala Ala Gly Leu
   130 135 140
   Leu Ala Leu Ala Ala Leu Ala Arg Gly Leu Gln Leu Ser Ala Leu Ala
   145 150 155 160
   Leu Ala Pro Ala Glu Val Gln Val Leu Arg Glu Ser Gly Ser Glu Ala
   165 170 175
   Glu Arg Ala Ala Ala Arg Arg Leu Glu Pro Ala Arg Arg Trp Ala Gly
   180 185 190
   Cys Ala Leu Gly Ala Leu Leu Leu Leu Ala Ser Leu Ala Gln Ala Ala
   195 200 205
   Leu Ala Val Leu Leu Tyr Arg Ala Ala Gly Gln Arg Ala Val Pro Ala
   210 215 220
   Val Leu Gly Ser Ala Gly Leu Val Phe Leu Val Gly Glu Val Val Pro
   225 230 235 240
   Ala Ala Val Ser Gly Arg Trp Thr Leu Ala Leu Ala Pro Arg Ala Leu
   245 250 255
   Gly Leu Ser Arg Leu Ala Val Leu Leu Thr Leu Pro Val Ala Leu Pro
   260 265 270
   Val Gly Gln Leu Leu Glu Leu Ala Ala Arg Pro Gly Arg Leu Arg Glu
   275 280 285
   Arg Val Leu Glu Leu Ala Arg Gly Gly Gly Asp Pro Tyr Ser Asp Leu
   290 295 300
   Ser Lys Gly Val Leu Arg Cys Arg Thr Val Glu Asp Val Leu Thr Pro
   305 310 315 320
   Leu Glu Asp Cys Phe Met Leu Asp Ala Ser Thr Val Leu Asp Phe Gly
   325 330 335
   Val Leu Ala Ser Ile Met Gln Ser Gly His Thr Arg Ile Pro Val Tyr
   340 345 350
   Glu Glu Glu Arg Ser Asn Ile Val Asp Met Leu Tyr Leu Lys Asp Leu
   355 360 365
   Ala Phe Val Asp Pro Glu Asp Cys Thr Pro Leu Ser Thr Ile Thr Arg
   370 375 380
   Phe Tyr Asn His Pro Leu His Phe Val Phe Asn Asp Thr Lys Leu Asp
   385 390 395 400
   Ala Val Leu Glu Glu Phe Lys Arg Gly Lys Ser His Leu Ala Ile Val
   405 410 415
   Gln Lys Val Asn Asn Glu Gly Glu Gly Asp Pro Phe Tyr Glu Val Leu
   420 425 430
   Gly Leu Val Thr Leu Glu Asp Val Ile Glu Glu Ile Ile Arg Ser Glu
   435 440 445
   Ile Leu Asp Glu Ser Glu Asp Tyr Arg Asp Thr Val Val Lys Arg Lys
   450 455 460
   Pro Ala Ser Leu Met Ala Pro Leu Lys Arg Lys Glu Glu Phe Ser Leu
   465 470 475 480
   Phe Lys Val Ser Asp Asp Glu Tyr Lys Val Thr Ile Ser Pro Gln Leu
   485 490 495
   Leu Leu Ala Thr Gln Arg Phe Leu Ser Arg Glu Val Asp Val Phe Ser
   500 505 510
   Pro Leu Arg Ile Ser Glu Lys Val Leu Leu His Leu Leu Lys His Pro
   515 520 525
   Ser Val Asn Gln Glu Val Arg Phe Asp Glu Ser Asn Arg Leu Ala Thr
   530 535 540
   His His Tyr Leu Tyr Gln Arg Ser Gln Pro Val Asp Tyr Phe Ile Leu
   545 550 555 560
   Ile Leu Gln Gly Arg Val Glu Val Glu Ile Gly Lys Glu Gly Leu Lys
   565 570 575
   Phe Glu Asn Gly Ala Phe Thr Tyr Tyr Gly Val Ser Ala Leu Thr Val
   580 585 590
   Pro Ser Ser Val His Gln Ser Pro Val Ser Ser Leu Gln Pro Ile Arg
   595 600 605
   His Asp Leu Gln Pro Asp Pro Gly Asp Gly Thr His Ser Ser Ala Tyr
   610 615 620
   Cys Pro Asp Tyr Thr Val Arg Ala Leu Ser Asp Leu Gln Leu Ile Lys
   625 630 635 640
   Val Thr Arg Leu Gln Tyr Leu Asn Ala Leu Leu Ala Thr Arg Ala Gln
   645 650 655
   Asn Leu Pro Gln Ser Pro Glu Asn Thr Asp Leu Gln Val Ile Pro Gly
   660 665 670
   Ser Gln Thr Arg Leu Leu Gly Glu Lys Thr Thr Thr Ala Ala Gly Ser
   675 680 685
   Ser His Ser Arg Pro Gly Val Pro Val Glu Gly Ser Pro Gly Arg Asn
   690 695 700
   Pro Gly Val
   705
<210> 27
   <211> 501
   <212> PRT
   <213> Homo sapiens
<400> 27
   Met Val Arg Lys Pro Val Val Ser Thr Ile Ser Lys Gly Gly Tyr Leu
   1               5                   10                  15
   Gln Gly Asn Val Asn Gly Arg Leu Pro Ser Leu Gly Asn Lys Glu Pro
               20                  25                  30
   Pro Gly Gln Glu Lys Val Gln Leu Lys Arg Lys Val Thr Leu Leu Arg
           35                  40                  45
   Gly Val Ser Ile Ile Ile Gly Thr Ile Ile Gly Ala Gly Ile Phe Ile
       50                  55                  60
   Ser Pro Lys Gly Val Leu Gln Asn Thr Gly Ser Val Gly Met Ser Leu
   65                  70                  75                  80
   Thr Ile Trp Thr Val Cys Gly Val Leu Ser Leu Phe Gly Ala Leu Ser
                   85                  90                  95
   Tyr Ala Glu Leu Gly Thr Thr Ile Lys Lys Ser Gly Gly His Tyr Thr
               100                 105                 110
   Tyr Ile Leu Glu Val Phe Gly Pro Leu Pro Ala Phe Val Arg Val Trp
           115                 120                 125
   Val Glu Leu Leu Ile Ile Arg Pro Ala Ala Thr Ala Val Ile Ser Leu
       130                 135                 140
   Ala Phe Gly Arg Tyr Ile Leu Glu Pro Phe Phe Ile Gln Cys Glu Ile
   145                 150                 155                 160
   Pro Glu Leu Ala Ile Lys Leu Ile Thr Ala Val Gly Ile Thr Val Val
                   165                 170                 175
   Met Val Leu Asn Ser Met Ser Val Ser Trp Ser Ala Arg Ile Gln Ile
               180                 185                 190
   Phe Leu Thr Phe Cys Lys Leu Thr Ala Ile Leu Ile Ile Ile Val Pro
           195                 200                 205
   Gly Val Met Gln Leu Ile Lys Gly Gln Thr Gln Asn Phe Lys Asp Ala
       210                 215                 220
   Phe Ser Gly Arg Asp Ser Ser Ile Thr Arg Leu Pro Leu Ala Phe Tyr
   225 230 235 240
   Tyr Gly Met Tyr Ala Tyr Ala Gly Trp Phe Tyr Leu Asn Phe Val Thr
   245 250 255
   Glu Glu Val Glu Asn Pro Glu Lys Thr Ile Pro Leu Ala Ile Cys Ile
   260 265 270
   Ser Met Ala Ile Val Thr Ile Gly Tyr Val Leu Thr Asn Val Ala Tyr
   275 280 285
   Phe Thr Thr Ile Asn Ala Glu Glu Leu Leu Leu Ser Asn Ala Val Ala
   290 295 300
   Val Thr Phe Ser Glu Arg Leu Leu Gly Asn Phe Ser Leu Ala Val Pro
   305 310 315 320
   Ile Phe Val Ala Leu Ser Cys Phe Gly Ser Met Asn Gly Gly Val Phe
   325 330 335
   Ala Val Ser Arg Leu Phe Tyr Val Ala Ser Arg Glu Gly His Leu Pro
   340 345 350
   Glu Ile Leu Ser Met Ile His Val Arg Lys His Thr Pro Leu Pro Ala
   355 360 365
   Val Ile Val Leu His Pro Leu Thr Met Ile Met Leu Phe Ser Gly Asp
   370 375 380
   Leu Asp Ser Leu Leu Asn Phe Leu Ser Phe Ala Arg Trp Leu Phe Ile
   385 390 395 400
   Gly Leu Ala Val Ala Gly Leu Ile Tyr Leu Arg Tyr Lys Cys Pro Asp
   405 410 415
   Met His Arg Pro Phe Lys Val Pro Leu Phe Ile Pro Ala Leu Phe Ser
   420 425 430
   Phe Thr Cys Leu Phe Met Val Ala Leu Ser Leu Tyr Ser Asp Pro Phe
   435 440 445
   Ser Thr Gly Ile Gly Phe Val Ile Thr Leu Thr Gly Val Pro Ala Tyr
   450 455 460
   Tyr Leu Phe Ile Ile Trp Asp Lys Lys Pro Arg Trp Phe Arg Ile Met
   465 470 475 480
   Ser Glu Lys Ile Thr Arg Thr Leu Gln Ile Ile Leu Glu Val Val Pro
   485 490 495
   Glu Glu Asp Lys Leu
   500
<210> 28
   <211> 905
   <212> PRT
   <213> Homo sapiens
<400> 28
<210> 29
   <211> 474
   <212> PRT
   <213> Homo sapiens
<400> 29
   Met Glu Leu Pro Ser Gly Pro Gly Pro Glu Arg Leu Phe Asp Ser His
   1               5                   10                  15
   Arg Leu Pro Gly Asp Cys Phe Leu Leu Leu Val Leu Leu Leu Tyr Ala
               20                  25                  30
   Pro Val Gly Phe Cys Leu Leu Val Leu Arg Leu Phe Leu Gly Ile His
           35                  40                  45
   Val Phe Leu Val Ser Cys Ala Leu Pro Asp Ser Val Leu Arg Arg Phe
       50                  55                  60
   Val Val Arg Thr Met Cys Ala Val Leu Gly Leu Val Ala Arg Gln Glu
   65                  70                  75                  80
   Asp Ser Gly Leu Arg Asp His Ser Val Arg Val Leu Ile Ser Asn His
                   85                  90                  95
   Val Thr Pro Phe Asp His Asn Ile Val Asn Leu Leu Thr Thr Cys Ser
               100                 105                 110
   Thr Val Ser Glu Ser Glu Ala Glu Ser Ala Thr Gly Arg Phe Pro Gly
           115                 120                 125
   Ala Gln Leu Lys Ala Pro Leu Ser Pro Leu Ala Phe Pro Met Glu Asp
       130                 135                 140
   Thr Glu Leu Pro Leu Thr Pro Ile Leu Tyr Pro Thr Cys Gln Phe Phe
   145                 150                 155                 160
   Phe Ile Phe Leu Asn Ile Phe Leu Leu Ala Phe Ser Ser Pro Gly Ser
                   165                 170                 175
   Gln Pro Leu Leu Asn Ser Pro Pro Ser Phe Val Cys Trp Ser Arg Gly
               180                 185                 190
   Phe Met Glu Met Asn Gly Arg Gly Glu Leu Val Glu Ser Leu Lys Arg
           195                 200                 205
   Phe Cys Ala Ser Thr Arg Leu Pro Pro Thr Pro Leu Leu Leu Phe Pro
       210                 215                 220
   Glu Glu Glu Ala Thr Asn Gly Arg Glu Gly Leu Leu Arg Phe Ser Ser
   225                 230                 235                 240
   Trp Pro Phe Ser Ile Gln Asp Val Val Gln Pro Leu Thr Leu Gln Val
                   245                 250                 255
   Gln Arg Pro Leu Val Ser Val Thr Val Ser Asp Ala Ser Trp Val Ser
               260                 265                 270
   Glu Leu Leu Trp Ser Leu Phe Val Pro Phe Thr Val Tyr Gln Val Arg
           275                 280                 285
   Trp Leu Arg Pro Val His Arg Gln Leu Gly Glu Ala Asn Glu Glu Phe
       290                 295                 300
   Ala Leu Arg Val Gln Gln Leu Val Ala Lys Glu Leu Gly Gln Thr Gly
   305 310 315 320
   Thr Arg Leu Thr Pro Ala Asp Lys Ala Glu His Met Lys Arg Gln Arg
   325 330 335
   His Pro Arg Leu Arg Pro Gln Ser Ala Gln Ser Ser Phe Pro Pro Ser
   340 345 350
   Pro Gly Pro Ser Pro Asp Val Gln Leu Ala Thr Leu Ala Gln Arg Val
   355 360 365
   Lys Glu Val Leu Pro His Val Pro Leu Gly Val Ile Gln Arg Asp Leu
   370 375 380
   Ala Lys Thr Gly Cys Val Asp Leu Thr Ile Thr Asn Leu Leu Glu Gly
   385 390 395 400
   Ala Val Ala Phe Met Pro Glu Asp Ile Thr Lys Gly Thr Gln Ser Leu
   405 410 415
   Pro Thr Ala Ser Ala Ser Lys Phe Pro Ser Ser Gly Pro Val Thr Pro
   420 425 430
   Gln Pro Thr Ala Leu Thr Phe Ala Lys Ser Ser Trp Ala Arg Gln Glu
   435 440 445
   Ser Leu Gln Glu Arg Lys Gln Ala Leu Tyr Glu Tyr Ala Arg Arg Arg
   450 455 460
   Phe Thr Glu Arg Arg Ala Gln Glu Ala Asp
   465 470
<210> 30
   <211> 532
   <212> PRT
   <213> Homo sapiens
<400> 30
<210> 31
   <211> 910
   <212> PRT
   <213> Homo sapiens
<400> 31
   Met Lys Lys Met Ser Arg Asn Val Leu Leu Gln Met Glu Glu Glu Glu
   1 5 10 15
   Asp Asp Asp Asp Gly Asp Ile Val Leu Glu Asn Leu Gly Gln Thr Ile
   20 25 30
   Val Pro Asp Leu Gly Ser Leu Glu Ser Gln His Asp Phe Arg Thr Pro
   35 40 45
   Glu Phe Glu Glu Phe Asn Gly Lys Pro Asp Ser Leu Phe Phe Asn Asp
   50 55 60
   Gly Gln Arg Arg Ile Asp Phe Val Leu Val Tyr Glu Asp Glu Ser Arg
   65 70 75 80
   Lys Glu Thr Asn Lys Lys Gly Thr Asn Glu Lys Gln Arg Arg Lys Arg
   85 90 95
   Gln Ala Tyr Glu Ser Asn Leu Ile Cys His Gly Leu Gln Leu Glu Ala
   100 105 110
   Thr Arg Ser Val Leu Asp Asp Lys Leu Val Phe Val Lys Val His Ala
   115 120 125
   Pro Trp Glu Val Leu Cys Thr Tyr Ala Glu Ile Met His Ile Lys Leu
   130 135 140
   Pro Leu Lys Pro Asn Asp Leu Lys Asn Arg Ser Ser Ala Phe Gly Thr
   145 150 155 160
   Leu Asn Trp Phe Thr Lys Val Leu Ser Val Asp Glu Ser Ile Ile Lys
   165 170 175
   Pro Glu Gln Glu Phe Phe Thr Ala Pro Phe Glu Lys Asn Arg Met Asn
   180 185 190
   Asp Phe Tyr Ile Val Asp Arg Asp Ala Phe Phe Asn Pro Ala Thr Arg
   195 200 205
   Ser Arg Ile Val Tyr Phe Ile Leu Ser Arg Val Lys Tyr Gln Val Ile
   210 215 220
   Asn Asn Val Ser Lys Phe Gly Ile Asn Arg Leu Val Asn Ser Gly Ile
   225 230 235 240
   Tyr Lys Ala Ala Phe Pro Leu His Asp Cys Lys Phe Arg Arg Gln Ser
   245 250 255
   Glu Asp Pro Ser Cys Pro Asn Glu Arg Tyr Leu Leu Tyr Arg Glu Trp
   260 265 270
   Ala His Pro Arg Ser Ile Tyr Lys Lys Gln Pro Leu Asp Leu Ile Arg
           275                 280                 285
   Lys Tyr Tyr Gly Glu Lys Ile Gly Ile Tyr Phe Ala Trp Leu Gly Tyr
       290                 295                 300
   Tyr Thr Gln Met Leu Leu Leu Ala Ala Val Val Gly Val Ala Cys Phe
   305                 310                 315                 320
   Leu Tyr Gly Tyr Leu Asn Gln Asp Asn Cys Thr Trp Ser Lys Glu Val
                   325                 330                 335
   Cys His Pro Asp Ile Gly Gly Lys Ile Ile Met Cys Pro Gln Cys Asp
               340                 345                 350
   Arg Leu Cys Pro Phe Trp Lys Leu Asn Ile Thr Cys Glu Ser Ser Lys
           355                 360                 365
   Lys Leu Cys Ile Phe Asp Ser Phe Gly Thr Leu Val Phe Ala Val Phe
       370                 375                 380
   Met Gly Val Trp Val Thr Leu Phe Leu Glu Phe Trp Lys Arg Arg Gln
   385                 390                 395                 400
   Ala Glu Leu Glu Tyr Glu Trp Asp Thr Val Glu Leu Gln Gln Glu Glu
                   405                 410                 415
   Gln Ala Arg Pro Glu Tyr Glu Ala Arg Cys Thr His Val Val Ile Asn
               420                 425                 430
   Glu Ile Thr Gln Glu Glu Glu Arg Ile Pro Phe Thr Ala Trp Gly Lys
           435                 440                 445
   Cys Ile Arg Ile Thr Leu Cys Ala Ser Ala Val Phe Phe Trp Ile Leu
       450                 455                 460
   Leu Ile Ile Ala Ser Val Ile Gly Ile Ile Val Tyr Arg Leu Ser Val
   465                 470                 475                 480
   Phe Ile Val Phe Ser Ala Lys Leu Pro Lys Asn Ile Asn Gly Thr Asp
                   485                 490                 495
   Pro Ile Gln Lys Tyr Leu Thr Pro Gln Thr Ala Thr Ser Ile Thr Ala
               500                 505                 510
   Ser Ile Ile Ser Phe Ile Ile Ile Met Ile Leu Asn Thr Ile Tyr Glu
           515                 520                 525
   Lys Val Ala Ile Met Ile Thr Asn Phe Glu Leu Pro Arg Thr Gln Thr
       530                 535                 540
   Asp Tyr Glu Asn Ser Leu Thr Met Lys Met Phe Leu Phe Gln Phe Val
   545                 550                 555                 560
   Asn Tyr Tyr Ser Ser Cys Phe Tyr Ile Ala Phe Phe Lys Gly Lys Phe
                   565                 570                 575
   Val Gly Tyr Pro Gly Asp Pro Val Tyr Trp Leu Gly Lys Tyr Arg Asn
               580                 585                 590
   Glu Glu Cys Asp Pro Gly Gly Cys Leu Leu Glu Leu Thr Thr Gln Leu
           595                 600                 605
   Thr Ile Ile Met Gly Gly Lys Ala Ile Trp Asn Asn Ile Gln Glu Val
       610                 615                 620
   Leu Leu Pro Trp Ile Met Asn Leu Ile Gly Arg Phe His Arg Val Ser
   625                 630                 635                 640
   Gly Ser Glu Lys Ile Thr Pro Arg Trp Glu Gln Asp Tyr His Leu Gln
                   645                 650                 655
   Pro Met Gly Lys Leu Gly Leu Phe Tyr Glu Tyr Leu Glu Met Ile Ile
               660                 665                 670
   Gln Phe Gly Phe Val Thr Leu Phe Val Ala Ser Phe Pro Leu Ala Pro
           675                 680                 685
   Leu Leu Ala Leu Val Asn Asn Ile Leu Glu Ile Arg Val Asp Ala Trp
       690                 695                 700
   Lys Leu Thr Thr Gln Phe Arg Arg Leu Val Pro Glu Lys Ala Gln Asp
   705                 710                 715                 720
   Ile Gly Ala Trp Gln Pro Ile Met Gln Gly Ile Ala Ile Leu Ala Val
                   725                 730                 735
   Val Thr Asn Ala Met Ile Ile Ala Phe Thr Ser Asp Met Ile Pro Arg
               740                 745                 750
   Leu Val Tyr Tyr Trp Ser Phe Ser Val Pro Pro Tyr Gly Asp His Thr
           755                 760                 765
   Ser Tyr Thr Met Glu Gly Tyr Ile Asn Asn Thr Leu Ser Ile Phe Lys
       770                 775                 780
   Val Ala Asp Phe Lys Asn Lys Ser Lys Gly Asn Pro Tyr Ser Asp Leu
   785                 790                 795                 800
   Gly Asn His Thr Thr Cys Arg Tyr Arg Asp Phe Arg Tyr Pro Pro Gly
                   805                 810                 815
   His Pro Gln Glu Tyr Lys His Asn Ile Tyr Tyr Trp His Val Ile Ala
               820                 825                 830
   Ala Lys Leu Ala Phe Ile Ile Val Met Glu His Val Ile Tyr Ser Val
           835                 840                 845
   Lys Phe Phe Ile Ser Tyr Ala Ile Pro Asp Val Ser Lys Arg Thr Lys
       850                 855                 860
   Ser Lys Ile Gln Arg Glu Lys Tyr Leu Thr Gln Lys Leu Leu His Glu
   865                 870                 875                 880
   Asn His Leu Lys Asp Met Thr Lys Asn Met Gly Val Ile Ala Glu Arg
                   885                 890                 895
   Met Ile Glu Ala Val Asp Asn Asn Leu Arg Pro Lys Ser Glu
               900                 905                 910
<210> 32
   <211> 879
   <212> PRT
   <213> Homo sapiens
<400> 32
   Met Gly Arg Leu Ala Ser Arg Pro Leu Leu Leu Ala Leu Leu Ser Leu
   1               5                   10                  15
   Ala Leu Cys Arg Gly Arg Val Val Arg Val Pro Thr Ala Thr Leu Val
               20                  25                  30
   Arg Val Val Gly Thr Glu Leu Val Ile Pro Cys Asn Val Ser Asp Tyr
           35                  40                  45
   Asp Gly Pro Ser Glu Gln Asn Phe Asp Trp Ser Phe Ser Ser Leu Gly
       50                  55                  60
   Ser Ser Phe Val Glu Leu Ala Ser Thr Trp Glu Val Gly Phe Pro Ala
   65                  70                  75                  80
   Gln Leu Tyr Gln Glu Arg Leu Gln Arg Gly Glu Ile Leu Leu Arg Arg
                   85                  90                  95
   Thr Ala Asn Asp Ala Val Glu Leu His Ile Lys Asn Val Gln Pro Ser
               100                 105                 110
   Asp Gln Gly His Tyr Lys Cys Ser Thr Pro Ser Thr Asp Ala Thr Val
   115 120 125
   Gln Gly Asn Tyr Glu Asp Thr Val Gln Val Lys Val Leu Ala Asp Ser
   130 135 140
   Leu His Val Gly Pro Ser Ala Arg Pro Pro Pro Ser Leu Ser Leu Arg
   145 150 155 160
   Glu Gly Glu Pro Phe Glu Leu Arg Cys Thr Ala Ala Ser Ala Ser Pro
   165 170 175
   Leu His Thr His Leu Ala Leu Leu Trp Glu Val His Arg Gly Pro Ala
   180 185 190
   Arg Arg Ser Val Leu Ala Leu Thr His Glu Gly Arg Phe His Pro Gly
   195 200 205
   Leu Gly Tyr Glu Gln Arg Tyr His Ser Gly Asp Val Arg Leu Asp Thr
   210 215 220
   Val Gly Ser Asp Ala Tyr Arg Leu Ser Val Ser Arg Ala Leu Ser Ala
   225 230 235 240
   Asp Gln Gly Ser Tyr Arg Cys Ile Val Ser Glu Trp Ile Ala Glu Gln
   245 250 255
   Gly Asn Trp Gln Glu Ile Gln Glu Lys Ala Val Glu Val Ala Thr Val
   260 265 270
   Val Ile Gln Pro Ser Val Leu Arg Ala Ala Val Pro Lys Asn Val Ser
   275 280 285
   Val Ala Glu Gly Lys Glu Leu Asp Leu Thr Cys Asn Ile Thr Thr Asp
   290 295 300
   Arg Ala Asp Asp Val Arg Pro Glu Val Thr Trp Ser Phe Ser Arg Met
   305 310 315 320
   Pro Asp Ser Thr Leu Pro Gly Ser Arg Val Leu Ala Arg Leu Asp Arg
   325 330 335
   Asp Ser Leu Val His Ser Ser Pro His Val Ala Leu Ser His Val Asp
   340 345 350
   Ala Arg Ser Tyr His Leu Leu Val Arg Asp Val Ser Lys Glu Asn Ser
   355 360 365
   Gly Tyr Tyr Tyr Cys His Val Ser Leu Trp Ala Pro Gly His Asn Arg
       370                 375                 380
   Ser Trp His Lys Val Ala Glu Ala Val Ser Ser Pro Ala Gly Val Gly
   385                 390                 395                 400
   Val Thr Trp Leu Glu Pro Asp Tyr Gln Val Tyr Leu Asn Ala Ser Lys
                   405                 410                 415
   Val Pro Gly Phe Ala Asp Asp Pro Thr Glu Leu Ala Cys Arg Val Val
               420                 425                 430
   Asp Thr Lys Ser Gly Glu Ala Asn Val Arg Phe Thr Val Ser Trp Tyr
           435                 440                 445
   Tyr Arg Met Asn Arg Arg Ser Asp Asn Val Val Thr Ser Glu Leu Leu
       450                 455                 460
   Ala Val Met Asp Gly Asp Trp Thr Leu Lys Tyr Gly Glu Arg Ser Lys
   465                 470                 475                 480
   Gln Arg Ala Gln Asp Gly Asp Phe Ile Phe Ser Lys Glu His Thr Asp
                   485                 490                 495
   Thr Phe Asn Phe Arg Ile Gln Arg Thr Thr Glu Glu Asp Arg Gly Asn
               500                 505                 510
   Tyr Tyr Cys Val Val Ser Ala Trp Thr Lys Gln Arg Asn Asn Ser Trp
           515                 520                 525
   Val Lys Ser Lys Asp Val Phe Ser Lys Pro Val Asn Ile Phe Trp Ala
       530                 535                 540
   Leu Glu Asp Ser Val Leu Val Val Lys Ala Arg Gln Pro Lys Pro Phe
   545                 550                 555                 560
   Phe Ala Ala Gly Asn Thr Phe Glu Met Thr Cys Lys Val Ser Ser Lys
                   565                 570                 575
   Asn Ile Lys Ser Pro Arg Tyr Ser Val Leu Ile Met Ala Glu Lys Pro
               580                 585                 590
   Val Gly Asp Leu Ser Ser Pro Asn Glu Thr Lys Tyr Ile Ile Ser Leu
           595                 600                 605
   Asp Gln Asp Ser Val Val Lys Leu Glu Asn Trp Thr Asp Ala Ser Arg
       610                 615                 620
   Val Asp Gly Val Val Leu Glu Lys Val Gln Glu Asp Glu Phe Arg Tyr
   625                 630                 635                 640
   Arg Met Tyr Gln Thr Gln Val Ser Asp Ala Gly Leu Tyr Arg Cys Met
                   645                 650                 655
   Val Thr Ala Trp Ser Pro Val Arg Gly Ser Leu Trp Arg Glu Ala Ala
               660                 665                 670
   Thr Ser Leu Ser Asn Pro Ile Glu Ile Asp Phe Gln Thr Ser Gly Pro
           675                 680                 685
   Ile Phe Asn Ala Ser Val His Ser Asp Thr Pro Ser Val Ile Arg Gly
       690                 695                 700
   Asp Leu Ile Lys Leu Phe Cys Ile Ile Thr Val Glu Gly Ala Ala Leu
   705                 710                 715                 720
   Asp Pro Asp Asp Met Ala Phe Asp Val Ser Trp Phe Ala Val His Ser
                   725                 730                 735
   Phe Gly Leu Asp Lys Ala Pro Val Leu Leu Ser Ser Leu Asp Arg Lys
               740                 745                 750
   Gly Ile Val Thr Thr Ser Arg Arg Asp Trp Lys Ser Asp Leu Ser Leu
           755                 760                 765
   Glu Arg Val Ser Val Leu Glu Phe Leu Leu Gln Val His Gly Ser Glu
       770                 775                 780
   Asp Gln Asp Phe Gly Asn Tyr Tyr Cys Ser Val Thr Pro Trp Val Lys
   785                 790                 795                 800
   Ser Pro Thr Gly Ser Trp Gln Lys Glu Ala Glu Ile His Ser Lys Pro
                   805                 810                 815
   Val Phe Ile Thr Val Lys Met Asp Val Leu Asn Ala Phe Lys Tyr Pro
               820                 825                 830
   Leu Leu Ile Gly Val Gly Leu Ser Thr Val Ile Gly Leu Leu Ser Cys
           835                 840                 845
   Leu Ile Gly Tyr Cys Ser Ser His Trp Cys Cys Lys Lys Glu Val Gln
       850                 855                 860
   Glu Thr Arg Arg Glu Arg Arg Arg Leu Met Ser Met Glu Met Asp
   865                 870                 875

## Description

### SUBJECT OF THE INVENTION

The present invention relates to contrast agents, diagnostic markers and methods for detecting colorectal adenocarcinoma.

### BACKGROUND OF THE INVENTION

Most cancers are epithelial in origin and arise through stepwise progression from normal cells, through dysplasia, into malignant cells that invade surrounding tissues and have metastatic potential. Colorectal cancer (CRC; also referred to as colon cancer or large bowl cancer) is one prominent type of cancer undergoing such tumour progression.

CRC includes cancerous growth in the colon, rectum and appendix. It is one of the most significant human cancers with an incidence of about 1,000,000 new cases worldwide every year. Thus, CRC is the third most common cancer and the fourth leading cause of cancer-related deaths in the world (the second leading cause in the Western world; reviewed, e.g. in Gryfe, R. et al. (1997) Curr. Probl. Cancer 21, 233-300; Petersen, G. M. et al (1999) Cancer 86, 2540-2550.

CRC is curable if diagnosed at an early stage (i.e. stage I) of tumour development. The five-year survival rate for early stage CRC is >90%. At this early stage, most patients have no phenotypic symptoms of the disease. Early detection can markedly improve chances of long-term survival, as the five-year survival rate for CRC at a late stage (i.e. stage IV) of tumour development is only <5%. More than 95% of the cases of CRC are manifested as adenocarcinomas (Muto, T. et al. (1975) Cancer 36, 2251-2270; Fearon, E. R. et al. (1990) Cell 61, 759 767).

In the past, screenings have been undertaken to identify genes which are implicated in the progression from low-risk colorectal adenomas to high-risk adenocarcinomas. To this end, genes were identified, the expression of which is either up- or down-regulated in colorectal adenocarcinomas versus adenomas (see e.g. Carvalho et al. (2009) Gut 58, 79-89).

Currently, colonoscopy is the standard screening modality for CRC having the highest sensitivity and specificity to detect colorectal tumours among techniques that are currently applied.

In Carrer et al. (2008), Eur J Cancer. 44(12):1761-9, the expression levels of chemokines, angiogenic and angiostatic factors and their receptors were determined in paired mucosal and tumour samples of patients with colorectal carcinoma and correlated with clinical and histological parameters by advanced multivariate analyses. Document WO02057450 discloses nucleic acid sequences that encode novel polypeptides; the invention further discloses therapeutic, diagnostic and research methods for diagnosis, treatment, and prevention of disorders involving any one of these novel human nucleic acids and proteins. In Yanamoto et al. (2007), Oral Oncol. 43(9):869-77, it is hypothesized that Epithelial adhesion molecule (EpCAM) would be a good molecular target for cancer gene therapy. In Curtet et al. (1988), Int J Cancer Suppl.2:126-32, Monoclonal antibodies (MAbs) 19-9 and 73-3 specific for human colon adenocarcinoma were labelled with a high number of gadolinium atoms. The NMR contrast agent Gd-25 DTPA-MAb 19-9 or 73-3 ([Gd] 17 mumole/kg, [MAb] 60 microM) was injected into nude mice bearing human colon adenocarcinoma (SW948). Imaging was performed with this model. Very good contrast was obtained 24 hr after Gd-25 DTPA-MAb injection. In Chang et al. (2005, Cancer Detect Prev 29(6):562-9, cytochrome CYP1B1 is evaluated as biomarker alone or in combination with aryl hydrocarbon receptor (AhR), nuclear beta-catenin, p53 or bcl-2 for early diagnosis and prevention of colorectal cancer. In Thierolf et al. (2008), Proteomics Clin Appl 2(1):11-22, the proteome of normal and malignant colonic tissue is characterized. In Hallak et al. (1998), Int J Oncol 12(4):785-91, a total number of 120 colorectal malignant tumor tissues has been investigated.

Nevertheless, screening by colonoscopy may have disadvantages. Among those are (i) that colonoscopy is an invasive technique being a limiting factor when CRC screening is offered for asymptomatic individuals, (ii) that there is a small but significant risk of bowel perforation as a consequence of colonoscopy and (iii) that colonoscopy cannot discriminate between non-progressive (low-risk) and progressive (high-risk) colon tumour lesions.

Thus, there is a continuing need for agents, compositions, markers and methods that allow diagnosis of CRC.

### OBJECTIVE AND SUMMARY OF THE INVENTION

It is one objective of the present disclosure to provide for methods that allow diagnosing CRC.

The present invention is concerned with the provision of contrast agents for use in the detection of CRC as defined in the claims.

These and other objectives as they will become apparent from the ensuing description hereinafter form the subject matter of the independent claims. Some of the preferred embodiments of the present invention form the subject matter of the dependent claims.

Yet another aspect of the present disclosure relates to a contrast agent, optionally for use in magnetic resonance imaging (MRI) and/or magnetic photon imaging (MPI) comprising at least at least one compound being capable of interacting with a polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or A polypeptide of SEQ ID No.: 32.

In a preferred embodiment such compounds are coupled to marker molecules which preferably are detectable by MRI or MPI.

The compounds may preferably be antibodies.

Such contrast agents may preferably be used for detecting CRC.

In a further embodiment the present disclosure relates to use of at least one antibody capable of interacting with a polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32
as a contrast agent, optionally suitable for MRI and/or MPI.

Such contrast agents may preferably be used for detecting CRC.

Further, an embodiment of the present disclosure relates to a method of identifying at least one target molecule suitable as diagnostic marker for colorectal cancer, wherein the method comprises at least the following steps:
a) Obtaining cells from different human individuals all suffering from colorectal cancer and/or obtaining colorectal cancer cell lines;
b) Labeling polypeptides on the surface of said cells;
c) Isolating labelled polypeptides from non-labelled polypeptides;
d) Identification of isolated labelled polypeptides from the cell surface polypeptide-fraction of step c),
e) Of the polypeptides of step d), selecting those isolated labelled polypeptides for which genomic expression data indicate an increased expression compared to cells obtained from healthy individuals;
f) Of the polypeptides selected in step e), further selecting those isolated labelled polypeptides which comprise at least one transmembrane domain and/or at least one signal peptide;
g) Of the polypeptides selected in step f), further selecting those polypeptides with a positive RSC;
h) Of the polypeptides selected in step g), further selecting those polypeptides found within at least 70% of tested cells from different human individuals;
i) Of the polypeptides selected in step h), further selecting those polypeptides for which histological analysis confirms localization to the plasma membrane.

The aforementioned diagnostic markers, contrast agents, uses and methods are suitable for differentiating progressive (high-risk) CRC (adenocarcinomas) from non-progressive (low-risk) colorectal adenomas.

Further, the diagnostic markers, contrast agents, uses and methods of the present disclosure may be particularly suitable for non-invasive molecular imaging, for instance by MRI, for diagnosis of CRC.

Other embodiments of the present disclosure will become apparent from the detailed description hereinafter.

### FIGURE LEGENDS

Fig. 1 depicts SDS-PAGE analysis of Neuravidine-purified biotin-labelled cell surface or intracellular fractions of colorectal cancer cell lines Colo 205, HT-29, Caco2, RKO and HCT116. The two lanes of intracellular fractions correspond to the fractions obtained from the CRC cell lines Colo 205, HT29 and Caco2 (left lane) and RKO and HCT116 (right lane).
Fig. 2 depicts histological analysis of PRNP (SEQ ID NO: 6).
Fig. 3 depicts histological detection of BCAM (SEQ ID NO: 5) in tissue culture obtained from colorectal cancer patients.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. In the context of the present invention, the terms "about" or "approximately" denote an interval of accuracy that the person skilled in the art will understand to still ensure the technical effect of the feature in question. The term typically indicates deviation from the indicated numerical value of ±10%, and preferably of ±5%.

Further definitions of terms will be given in the following in the context of which the terms are used.

As mentioned in the background section, identification of molecular marker (patterns) for diagnosing CRC has focussed on the identification of genes the expression of which is either up- or down-regulated during CRC development. While such data provides valuable information, the diagnostic markers, i.e. genes and their products (e.g. polypeptides) may not all be suitable for non-invasive molecular imaging diagnostic approaches.

The inventors of the present invention have succeeded in identifying a set of polypeptides which are likely to be over-expressed in the majority of patients suffering from colorectal cancer development versus healthy individuals and which may be accessible for non-invasive molecular imaging diagnostic methods.

To this end, the inventors have devised a screening strategy comprising at least the following steps:
a) Obtaining cells from different human individuals all suffering from colorectal cancer and/or obtaining colorectal cancer cell lines;
b) Labeling polypeptides on the surface of said cells;
c) Isolating labelled polypeptides from non-labelled polypeptides;
d) Identification of isolated labelled polypeptides from the cell surface polypeptide-fraction of step c),
e) Of the polypeptides of step d), selecting those isolated labelled polypeptides for which genomic expression data indicate an increased expression compared to cells obtained from healthy individuals;
f) Of the polypeptides selected in step e), further selecting those isolated labelled polypeptides which comprise at least one transmembrane domain and/or at least one signal peptide;
g) Of the polypeptides selected in step f), further selecting those polypeptides with a positive RSC;
h) Of the polypeptides selected in step g), further selecting those polypeptides found within at least 70% of tested cells from different human individuals;
i) Of the polypeptides selected in step h), further selecting those polypeptides for which histological analysis confirms localization to the plasma membrane.

The cells of step a) which are obtained from human individuals suffering from CRC will typically be cell types that are known to be involved in the development of CRC. Typically, the cells will comprise embryonic, fetal or adult stem cells; progenitor cells; blood cells such as B and T-lymphocytes, monocytes and macrophages; epithelial cells; fibroblasts; and neuronal cells. Epithelial cells may be preferred.

The cells used in step a) may also be established CRC cell lines. Such cell lines are commercially available and include *inter alia* Colo205, HT-29, Caco-2, RKO, HCT116, SW1398, LS513, SW480, SW620 and COLO 320..

The labeling step b) is undertaken to allow for efficient purification, enrichment and isolation of preferentially polypeptides that localise to the surface of cells. Thus, one will use labels which allow for efficient purification, e.g. by affinity chromatography. Such labels may include e.g. biotin, maltose binding protein (MBP), Glutathione-S-transferase (GST), histidine-tags, Flag-tags, antibodies and the like.

The labels may be covalently or non-covalently attached to proteins being preferentially located on the cell surface.

For covalent modification one may use coupling chemistries that are commonly used for such purposes. Thus, one may use homo and/or hetero-bi and/or multifunctional crosslinking agents. Typical cross-linking agents include but are not limited to bis (sulfosuccinimid) bis (diazo-benzidine), Dimethyl Adipimidate, Dimethyl Pimelimidate, Dimethyl Suberaimidate, Disuccininnclyl Suberate, Glutaraldehyde, in-Maleimidobenzoyl-N-Hydroxysuccinimide, Sulfosuccinidyl 4-(N-Maleimidomethyl) Cyclohexane-1-carboxylate etc.

A preferred label may be sulfo-NHS-SS-biotin.

Subsequently, the cells are typically lysed. This may be achieved by hypotonic, enzymatic, ultrasound or mechanical cell rupture.

Subsequently, labelled polypeptides may be isolated making use of the label. The person skilled in the art knows how to select the appropriate isolation procedure.

If for example a biotin-label is used for labelling polypeptides, streptavidine- based chromatographic systems such as streptavidine-labelled or neuravidine-labelled beads as they are commercially available can be used. For His-tags, Ni-NTA agarose as available from Qiagen may be used. For Glutathion-S-transferase labels, GST-sepharose may be used etc.

By subjecting labelled polypeptides from e.g. lysed cells to such chromatographic media, purification of labelled polypeptides can be achieved. As the labelling procedure will typically preferentially label cell surface polypeptides, the fraction retained on and eluted from the chromatographic media may be designated as labelled cell surface polypeptide fraction. The polypeptides found in the flow through may be designated as the intracellular polypeptide fraction. Further, special preparation protocols as they are commonly known may be used to obtain fractions from the lysed cells comprising primarily cell surface associated polypeptides and intracellular polypeptides. Subjecting such fractions to chromatographic media as described above may improve adherence of labelled cell surface polypeptides to the chromatographic media.

If for example cells have been labelled with biotin, polypeptides of the cell will be preferentially labelled over cytoplasmic polypeptides such that labelled cell surface polypeptide will be preferentially retained on e.g. streptavidine-beads while cytoplasmic will be primarily found in the flow through or wash fractions.

The person skilled in the art is aware how to correctly perform these isolation/purification procedures, e.g. how to select suitable washing buffers, elution buffers, pH-values etc.

The labelled polypeptides from the cell surface polypeptide-fraction will thus be isolated by eluting them form the respective chromatographic media.

Subsequently, such isolated labelled polypeptides will be identified. For this, one may use e.g. mass spectrometry (MS) analysis.

The data obtained by this procedure will then be correlated with genomic expression data on genes, the expression of which is up- or down-regulated in CRC. Genomic expression data may be obtained from e.g. Carvalho et al. (2009) Gut 58 (1), 79-89 .

Correlation in the context of the present disclosure means that only those identified labelled polypeptides will be selected for which genomic expression data indicate an increased expression compared to cells obtained from healthy individuals.

In order to increase the likelihood that the labelled, isolated and identified polypeptides indeed localise to the plasma membrane of cells, a further selection criteria is applied which selects only those polypeptides for which an algorithm-based analysis indicates the presence of a transmembrane (TM) domain and/or at least one signal peptide. The term "signal peptide" refers to sequence signatures that direct localization of proteins to the plasma membrane of cells. Such signal peptide sequences are known in the art.

This analysis may be conducted using programmes and algorithms commonly used for such approaches such as Phobias developed by the Stockholm Bioinformatics Center (Käll et al. (2004) Journal of Molecular Biology, 338(5):1027-1036).

After selection of polypeptides with at least one transmembrane domain or at least one signal peptide, a further selection is applied to reduce the number of false positives. To this end, one selects only such proteins with a positive relative count (RSC) value.

In such an analysis, one determines which labelled polypeptides are enriched in the cell surface polypeptide fraction (i.e. preferentially retained on the chromatographic media) when compared to the intracellular polypeptide fraction. A log2 ratio measured from spectral counts (RSC) analysis is then performed. In principle, this analysis relies on a labelfree quantification approach based on spectral counts and is suitable to calculate relative protein abundance between samples. A detailed description can be found in Old et al (2005) Molecular & Cellular Proteomics 4(10): 1487-1502.

Using this analysis, only proteins with a positive RSC value are then selected.

The polypeptides identified by this series of different steps are then further analysed by identifying those polypeptides found within at least 70% of cells tested in step a) As pointed out above, the cells may be either obtained from different human individuals or may be established CRC cell lines. Typically, one will analyse at least five different cells meaning that e.g. at least cells from five different human individuals all suffering from CRC or at least five different CRC cell lines are analysed.

In a last selection step, polypeptides are selected for which histological analysis confirms localisation at least to the plasma membrane. Such histological analysis may be undertaken using e.g. antibodies being specific for the selected polypeptides.

Typically, the histological analysis will involve immunofluorescent analysis of cells obtained either from human individuals suffering from CRC or of established CRC cell lines. The specific protocols used for such histological analysis will usually depend on the specific polypeptide analysed. Nevertheless, the person skilled in the art will be able to rely on knowledge generally available for e.g. immunofluorescent detection and localisation of polypeptides.

Basically, such approaches will involve fixation of cells on a solid support such as a transparent microscopic cover slide. Subsequently, the cells are lysed and fixed before labelling with e.g. antibodies is performed.

Such approaches are described *inter alia* in Carvalho et al. (2009) Gut 58 (1), 79-89.

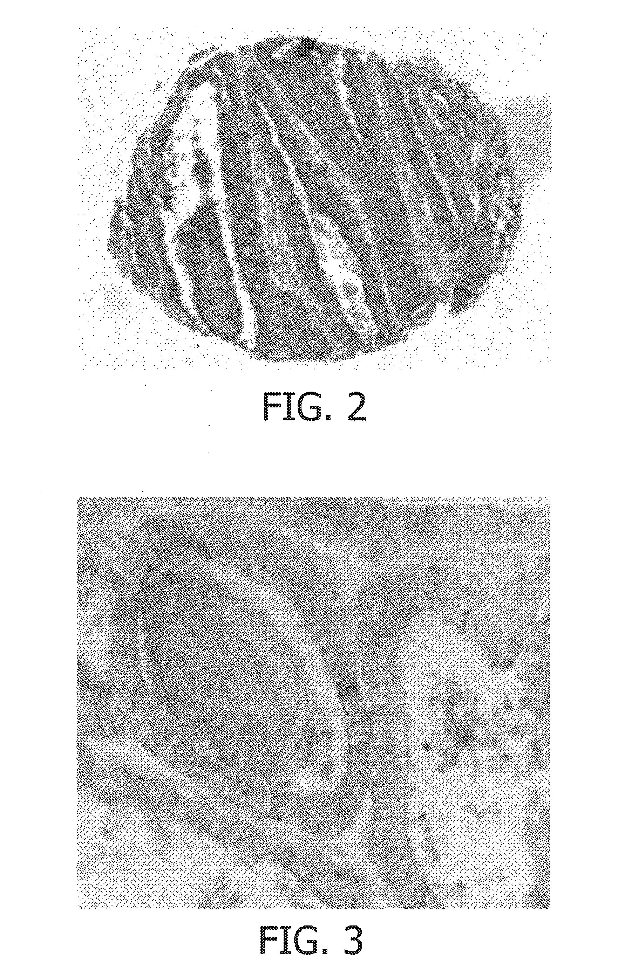

The inventors of the present invention relying on the steps a) to step h) have succeeded in identifying a set of 32 proteins which fulfil the aforementioned criteria. Further, histological analysis of PRNP (SEQ ID NO: 6) and BCAM (SEQ ID NO: 5) confirmed that these two proteins indeed localise at least to the plasma membrane (see Figure 2 and Figure 3, respectively).

A summary of the properties of these 32 proteins can be taken from Table 1.

**Table 1**

| Protein name | SEQ ID No: | TM region | Protein MW | cell line count | RSC | DIFF IN EXPRESSION |
|---|---|---|---|---|---|---|
| PLXNA1 Plexin-A1 precursor | 1 | 1 | 211071,9 | 5 | 5,972006932 | -0,616236226 |
| SLC1A5 Neutral amino acid transporter B | 2 | 9 | 56581,8 | 5 | 2,653686031 | -0,292097734 |
| SCARB1 Isoform 1 of Scavenger receptor class B member 1 | 3 | 1 | 56956,6 | 5 | 4,256484988 | -0,139340706 |
| ICAM1 Intercellular adhesion molecule 1 precursor | 4 | 1 | 57806,5 | 5 | 4,688794738 | -0,502549651 |
| BCAM Lutheran blood group glycoprotein precursor | 5 | 1 | 67385,9 | 5 | 6,546313113 | -0,511112343 |
| PRNP Major prion protein precursor | 6 | 2 | 27642,7 | 5 | 4,756670474 | -0,549604991 |
| DAG1 Dystroglycan precursor | 7 | 1 | 97563,3 | 5 | 6,54021969 | -0,248052408 |
| PTGFRN Prostaglandin F2 receptor negative regulator precursor | 8,32 | 1 | 98537,5 | 4 | 6,027860921 | -0,298927655 |
| IGSF8 Isoform 1 of Immunoglobulin superfamily member 8 precursor | 9 | 1 | 65014,8 | 5 | 4,376764152 | -0,32307837 |
| LDLR Low-density lipoprotein receptor precursor | 10 | 1 | 95356,7 | 5 | 5,046917378 | -0,409453599 |
| SLC1A3 Excitatory amino acid transporter 1 | 11 | 7 | 59555,8 | 4 | 3,673690871 | -0,56633461 |
| ACSL1 Isoform 1 of Long-chain-fatty-acid-CoA ligase 1 | 12 | 1 | 77927,1 | 4 | 2,494870467 | -0,468910389 |
| ITFG3 69 kDa protein | 13 | 1 | 69329,2 | 5 | 3,369073363 | -0,537911898 |
| LRP8 Isoform 1 of Low-density lipoprotein receptor-related protein 8 precursor | 14 | 1 | 105658,4 | 4 | 1,495012337 | -0,447612397 |
| CYB5B cytochrome b5 outer mitochondrial membrane precursor | 15 | 1 | 16676,8 | 5 | 3,31042916 | -0,40857396 |
| COPS6 COP9 signalosome complex subunit 6 | 16 | 1 | 36145,4 | 5 | 1,0369939 | -0,243432062 |
| PRSS8 Prostasin precursor | 17 | 2 | 36745,6 | 4 | 4,462248084 | -0,603233346 |
| SDC4 Syndecan-4 precursor | 18 | 1 | 21624,1 | 4 | 2,331824104 | -0,636360718 |
| SLC2A1 Solute carrier family 2, facilitated glucose transporter member 1 | 19 | 12 | 54067,3 | 5 | 2,144242719 | -0,65733993 |
| BST2 Bone marrow stromal antigen 2 precursor | 20 | 1 | 19751,3 | 4 | 3,045513157 | -0,902767067 |
| HSD17B7 Isoform 1 of 3-keto-steroid reductase | 21 | 1 | 38189,9 | 5 | 2,505469898 | -0,19805186 |
| CPOX Coproporphyrinogen III oxidase, mitochondrial precursor | 22 | 1 | 50133,9 | 5 | 1,673454923 | -0,22411626 |
| PODXL Podocalyxin-like protein 1 precursor | 23 | 1 | 55577,7 | 4 | 2,807508469 | -0,376649598 |
| DHCR7 7-dehydrocholesterol reductase | 24 | 7 | 54473 | 5 | 1,858119548 | -0,350599051 |
| SLC4A2 Isoform A of Anion exchange protein 2 | 25 | 13 | 136994,4 | 4 | 4,5335838 | -0,284851412 |
| CNNM3 cyclin M3 isoform 1 | 26 | 3 | 76102,5 | 4 | 2,904753941 | -0,126520099 |
| SLC7A11 Cystine/glutamate transporter | 27 | 14 | 55407,5 | 5 | 3,275370303 | -0,20939536 |
| KIAA1524 Isoform 1 of Protein KIAA1524 | 28 | 1 | 102171,3 | 4 | 2,419056717 | -0,281066707 |
| AUP1 Isoform Long of Ancient ubiquitous protein 1 precursor | 29 | 2 | 53010,8 | 4 | 2,526552401 | -0,187455693 |
| SLC1A4 Neutral amino acid transporter A | 30 | 9 | 55706,4 | 4 | 2,369591427 | -0,186930822 |
| TMEM16F Transmembrane protein 16F | 31 | 8 | 106151,4 | 5 | 3,310893761 | -0,205564206 |

| | | | | | | |
|---|---|---|---|---|---|---|
| "Tm" indicates the number of transmembrane domains."Protein MW" indicates the theoretic molecular weight. "Cell line count" indicates in how many of the five CRC cell lines Colo205, Caco2, HT29, RKO and HCT 116 the respective polypeptide was observed. "RSC" indicates the RSC value measured. "DIFF IN EXPRESSION" indicates the difference by which the gene is overexpressed in CRC based on the data of Carvalho et al. (2009) Gut 58 (1). | | | | | | |

By applying even stricter selection criteria (namely using an RSC of at least 2.5, a set of 11 proteins emerged which may be particularly useful as diagnostic markers, or targets for contrast agents. This list of 11 polypeptides is shown in Table 2.

**Table 2**

| Protein name | SEQ ID No: | TM region | Protein MW | cell line count | RSC | DIFF IN EXPRESSION |
|---|---|---|---|---|---|---|
| PLXNA1 Plexin-A1 precursor | 1 | 1 | 211071,9 | 5 | 5,972006932 | -0,616236226 |
| SLC1A5 Neutral amino acid transporter B | 2 | 9 | 56581,8 | 5 | 2,653686031 | -0,292097734 |
| SCARB1 Isoform 1 of Scavenger receptor class B member 1 | 3 | 1 | 56956,6 | 5 | 4,256484988 | -0,139340706 |
| ICAM1 Intercellular adhesion molecule 1 precursor | 4 | 1 | 57806,5 | 5 | 4,688794738 | -0,502549651 |
| BCAM Lutheran blood group glycoprotein precursor | 5 | 1 | 67385,9 | 5 | 6,546313113 | -0,511112343 |
| PRNP Major prion protein precursor | 6 | 2 | 27642,7 | 5 | 4,756670474 | -0,549604991 |
| DAG1 Dystroglycan precursor | 7 | 1 | 97563,3 | 5 | 6,54021969 | -0,248052408 |
| PTGFRN Prostaglandin F2 receptor negative regulator precursor | 8,32 | 1 | 98537,5 | 4 | 6,027860921 | -0,298927655 |
| IGSF8 Isoform 1 of Immunoglobulin superfamily member 8 precursor | 9 | 1 | 65014,8 | 5 | 4,376764152 | -0,32307837 |
| LDLR Low-density lipoprotein receptor precursor | 10 | 1 | 95356,7 | 5 | 5,046917378 | -0,409453599 |

| | | | | | | |
|---|---|---|---|---|---|---|
| Tm" indicates the number of transmembrane domains."Protein MW" indicates the theoretic molecular weight."Cell line count" indicates in how many of the five CRC cell lines Colo205, Caco2, HT29, RKO and HCT 116 the respective polypeptide was observed. "RSC" indicates the RSC value measured. "DIFF IN EXPRESSION" indicates the difference by which the gene is overexpressed in CRC based on the data of Carvalho et al. (2009) Gut 58 (1) (***Is this correct? YES***). | | | | | | |

Accordingly, one embodiment of the present disclosure relates to a diagnostic marker, preferably for detecting CRC comprising at least one polypeptide of the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32.

The term "diagnostic marker" refers to the property of polypeptides of SEQ ID No. 1 to 32 as being suitable as a marker for detecting cancers such as CRC development. Thus, detection of at least one polypeptide of SEQ ID No. 1 to SEQ ID No. 32 will, in principle, allow one to decide whether a human individual for which over-expression of such a polypeptide in comparison to a suitable control has been shown suffers from e.g. CRC.

The person skilled in the art will be aware that the simultaneous detection of more than at least one polypeptide of SEQ ID No. 1 to 32 will increase the correctness of the prediction for the occurrence of e.g. CRC.

The present disclosure therefore also relates in a preferred embodiment to a diagnostic marker or a set of diagnostic markers comprising at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32.

A preferred diagnostic marker in accordance with the present disclosure relates to a diagnostic marker or a set of diagnostic markers comprising at least ten polypeptides of the group consisting of SEQ ID No. 1 to 10 and SEQ ID No. 32.

A further preferred diagnostic marker relates to a diagnostic marker or a set of diagnostic markers comprising at least the polypeptides of SEQ ID Nos.1, 2 and 3.

Yet another preferred diagnostic marker of the present disclosure relates to a diagnostic marker or a set of diagnostic markers comprising at least the polypeptides of SEQ ID No. 1, 2, 3 and 4.

The present disclosure also relates to the use of at least one polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32;
as a diagnostic marker for detecting CRC.

The present disclosure also relates to the use of at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32 as diagnostic markers for detecting CRC.

Preferably the disclosure relates to the use of at least ten polypeptides of the group consisting of SEQ ID No. 1 to 10 and SEQ ID No.: 32 as a diagnostic marker for detecting CRC.

Another preferred embodiment relates to the use of the polypeptides of SEQ ID Nos. 1, 2 and 3 as a diagnostic marker for detecting CRC.

Yet another preferred embodiment relates to to the use of the polypeptides of SEQ ID Nos. 1, 2, 3 and 4 as a diagnostic marker for detecting CRC.

In a preferred embodiment, the polypeptides selected from the group consisting of SEQ ID No. 1 to SEQ ID No. 32 and the above mentioned preferred combinations of polypeptides such as e.g. polypeptides with SEQ ID Nos. 1 to 10 and SEQ ID No.: 32, polypeptides of SEQ ID Nos. 1, 2 and 3, or polypeptides of SEQ ID Nos. 1, 2, 3 and 4 will be used in a diagnostic approach outside the human or animal body.

Another embodiment of the present disclosure uses at least one polypeptide selected from the group consisting of SEQ ID Nos: 1 to 32 or at least the aforementioned preferred combinations in a diagnostic approach that allows for online detection of the diagnostic marker within a human individual. Such a diagnostic approach may rely on magnetic resonance imaging (MRI) and/or magnetic photon resonance imaging (MPI). However, other approaches which allow the imaging of the presence of at least one polypeptide selected from the group consisting of SEQ ID Nos. 1 to 32 in order to detect CRC may also be applied.

Thus, in one embodiment the present disclosure relates to a contrast agent being capable of specifically detecting at least polypeptide selected from the group consisting of SEQ ID Nos. 1 to 32.

The term "contrast agent" and its grammatical variations refers to a molecular compound that is capable of specifically interacting with a polypeptide of SEQ ID Nos. 1 to 32 and which can be detected by an apparatus positioned outside the human or animal body. Preferably, such contrast agents are suitable for use in magnetic resonance imaging (MRI) or magnetic photon imaging (MPI).

The term "specifically interacting" and its grammatical variations refer to the property of a molecular compound to preferentially interact with a polypeptide of SEQ ID Nos. 1 to 32 on the cell surface of cells being present within the human or animal body over other proteins that are expressed by such cells.

Preferred contrast agents which may also be designated as contrast agent compositions will be capable of specifically detecting at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32. Other preferred contrast agents/compositions will be capable of detecting the group of polypeptides consisting of SEQ ID No.: 1 to 10 and SEQ ID No.: 32, the group of SEQ ID Nos. 1, 2 and 3 or the group of SEQ ID Nos. 1, 2, 3 and 4.

As MRI and MPI in principle allow for non-invasive molecular imaging, contrast agents which comprise compounds that are capable of specifically interacting with at least one polypeptide of SEQ ID Nos. 1 to 32 and preferably with the aforementioned groups and which can be detected by MRI and/or MPI are preferred.

A class of molecules which may be particularly suitable as contrast agents for the purposes of the present invention are antibodies.

Antibodies being specific for polypeptides of SEQ ID Nos. 1 to 32 and preferably for the aforementioned groups of polypeptides may be already commercially available such as Primary anti-prion, mouse clone 8H4 antibody from Sigma-Aldrich (St. Louis,MO,USA) (for SEQ ID No.6), mouse anti human CD239 from abD serotech (Oxford,UK) (for SEQ ID No. 5).

If antibodies specifically recognising polypeptides of SEQ ID Nos. 1 to 32 are not already available, they can be produced by methods known to the person skilled in the art. Such antibodies may be polyclonal or monoclonal antibodies. Monoclonal antibodies may be produced by classical hybridoma fusion technology or e.g. phage-display systems.

The term "antibody" is used in the context of the present invention in its common sense. However, in addition the term further includes antibody variants and derivatives such as a single chain antibody, a Fab-fragment, a Fab2-fragment, a multispecific antibody, a diabody, a triabody, a tetrabody, a minibody, a linear antibody, a chelating recombinant antibody, a tribody, a bibody, an intrabody, a nanobody, a small modular immunopharmaceutical (SMIP), a binding-domain immunoglobulin fusion protein, a camelized antibody, a V_{HH} containing antibody and the like.

Preferred embodiments of the present disclosure relate to contrast agents which comprise antibodies that interact at least with polypeptides of SEQ ID Nos. 1 to 10 and SEQ ID No.: 32, with polypeptides of SEQ ID Nos. 1, 2 and 3 or with polypeptides of SEQ ID Nos. 1, 2, 3 and 4.

Contrast agents comprising such antibodies may be provided in a form where the contrast agent is a composition comprising different sets of antibody with each antibody recognising a polypeptide of a specific SEQ ID. Thus, a contrast agent may comprise three antibodies recognising polypeptides of SEQ ID Nos. 1, 2 and 3, respectively.

However, contrast agents in accordance with the present disclosure may also comprise antibodies which are multi-specific. Thus, a contrast agent may comprise a single antibody which recognises e.g. the polypeptide of SEQ ID No. 1, 2 and 3, at the same time. Further, contrast agents in accordance with the present disclosure may comprise antibodies all of which recognise a polypeptide of the same SEQ ID. Thus, a contrast agent in accordance with the present disclosure may comprise e.g. three antibodies all of which are specific for SEQ ID No. 1 and e.g. four antibodies all of which are specific for SEQ ID No. 2 etc.

Contrast agents, aside from their property of being capable of specifically recognising polypeptides of SEQ ID Nos. 1 to 32 will in addition typically comprise a marker molecule which is detectable by the specific detection technology used.

For example, if fluorescent spectroscopy is used as a detection means, such marker molecules may comprise fluorophores as detectable marker molecules that can be excitated at a specific wavelength.

With respect to preferred contrast agents in accordance with the invention that are suitable for MRI, the contrast agents such as the above described antibodies may comprise a marker molecule which is detectable by MRI. Such detectable labels include e.g. USPIOS and 19Fluor.

Another embodiment of the present disclosure relates to the use of at least one antibody capable of interacting with a polypeptide selected from the group consisting of:
- A polypeptide of SEQ ID No.: 1;
- A polypeptide of SEQ ID No.: 2;
- A polypeptide of SEQ ID No.: 3;
- A polypeptide of SEQ ID No.: 4;
- A polypeptide of SEQ ID No.: 5;
- A polypeptide of SEQ ID No.: 6;
- A polypeptide of SEQ ID No.: 7;
- A polypeptide of SEQ ID No.: 8;
- A polypeptide of SEQ ID No.: 9;
- A polypeptide of SEQ ID No.: 10;
- A polypeptide of SEQ ID No.: 11;
- A polypeptide of SEQ ID No.: 12;
- A polypeptide of SEQ ID No.: 13;
- A polypeptide of SEQ ID No.: 14;
- A polypeptide of SEQ ID No.: 15;
- A polypeptide of SEQ ID No.: 16;
- A polypeptide of SEQ ID No.: 17;
- A polypeptide of SEQ ID No.: 18;
- A polypeptide of SEQ ID No.: 19;
- A polypeptide of SEQ ID No.: 20;
- A polypeptide of SEQ ID No.: 21;
- A polypeptide of SEQ ID No.: 22;
- A polypeptide of SEQ ID No.: 23;
- A polypeptide of SEQ ID No.: 24;
- A polypeptide of SEQ ID No.: 25;
- A polypeptide of SEQ ID No.: 26;
- A polypeptide of SEQ ID No.: 27;
- A polypeptide of SEQ ID No.: 28;
- A polypeptide of SEQ ID No.: 29;
- A polypeptide of SEQ ID No.: 30;
- A polypeptide of SEQ ID No.: 31; and/or
- A polypeptide of SEQ ID No.: 32
as a contrast agent suitable for MRI.

The invention also relates to the use of disclosure which interact with at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty, at least twenty one, at least twenty two, at least twenty three, at least twenty four, at least twenty five, at least twenty six, at least twenty seven, at least twenty eight, at least twenty nine, at least thirty or thirty one polypeptides of the group consisting of SEQ ID Nos. 1 to 32 as contrast agents.

Preferably the disclosure relates to the use of antibodies which interact with at least ten polypeptides of the group consisting of SEQ ID No. 1 to 10 and SEQ ID No.: 32 as contrast agents.

Another preferred embodiment relates to the use of antibodies which interact with the polypeptides of SEQ ID Nos. 1, 2 and 3 as contrast agents.

Yet another preferred embodiment relates to the use of antibodies which interact with the polypeptides of SEQ ID Nos. 1, 2, 3 and 4 as contrast agents.

As pointed out above, the diagnostic markers and contrast agents of the present disclosure may be used for detection and diagnosis of colorectal cancer. These methods may be used either outside or inside the human or animal body. Thus, one may e.g. use an antibody which is labelled to a fluorophore in the histological analysis for detecting polypeptides of SEQ ID Nos. 1 to 32 in cellular tissue and samples which have been obtained from an individual suspected of suffering from CRC.

In addition or alternatively, one may use e.g. antibodies for non-invasive molecular imaging techniques such as MRI.

As the diagnostic markers and contrast agents as described above are primarily used for the detection and diagnosis of CRC, one embodiment of the present disclosure relates to a method of diagnosing CRC comprising at least the following steps:
a) Obtaining at least one sample from at least one human or animal individual suspected to suffer from ongoing or imminent CRC development;
b) Testing in said at least one sample for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
c) Testing in at least one control sample obtained from at least one human or animal individual not suffering from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31
   - A polypeptide of SEQ ID No.: 32;
d) Determining difference in expression of steps b) and d);
e) Deciding on the presence or imminence of CRC development based on the results obtained in step d).

In one embodiment, steps b), c), d) and/or e) of this method of diagnosis are performed outside the human or animal body.

Another embodiment of the present disclosure relates to a method of diagnosing CRC comprising at least the following steps:
a) Testing in at least one human or animal individual suspected to suffer from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31
   - A polypeptide of SEQ ID No.: 32;
b) Comparing expression as determined in step a) with expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
      as determined for human or animal individuals not suffering from ongoing or imminent colorectal cancer development.
c) Deciding on the presence or imminence of colorectal cancer development based on the results obtained in step b).

Further, the present disclosure may relate to a method of data acquisition comprising at least the following steps:
a) Testing in at least one human or animal individual suspected to suffer from ongoing or imminent CRC development for expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
b) Comparing expression as determined in step a) with expression of at least one polypeptide selected from the group consisting of:
   - A polypeptide of SEQ ID No.: 1;
   - A polypeptide of SEQ ID No.: 2;
   - A polypeptide of SEQ ID No.: 3;
   - A polypeptide of SEQ ID No.: 4;
   - A polypeptide of SEQ ID No.: 5;
   - A polypeptide of SEQ ID No.: 6;
   - A polypeptide of SEQ ID No.: 7;
   - A polypeptide of SEQ ID No.: 8;
   - A polypeptide of SEQ ID No.: 9;
   - A polypeptide of SEQ ID No.: 10;
   - A polypeptide of SEQ ID No.: 11;
   - A polypeptide of SEQ ID No.: 12;
   - A polypeptide of SEQ ID No.: 13;
   - A polypeptide of SEQ ID No.: 14;
   - A polypeptide of SEQ ID No.: 15;
   - A polypeptide of SEQ ID No.: 16;
   - A polypeptide of SEQ ID No.: 17;
   - A polypeptide of SEQ ID No.: 18;
   - A polypeptide of SEQ ID No.: 19;
   - A polypeptide of SEQ ID No.: 20;
   - A polypeptide of SEQ ID No.: 21;
   - A polypeptide of SEQ ID No.: 22;
   - A polypeptide of SEQ ID No.: 23;
   - A polypeptide of SEQ ID No.: 24;
   - A polypeptide of SEQ ID No.: 25;
   - A polypeptide of SEQ ID No.: 26;
   - A polypeptide of SEQ ID No.: 27;
   - A polypeptide of SEQ ID No.: 28;
   - A polypeptide of SEQ ID No.: 29;
   - A polypeptide of SEQ ID No.: 30;
   - A polypeptide of SEQ ID No.: 31; and/or
   - A polypeptide of SEQ ID No.: 32;
as determined for human or animal individuals not suffering from ongoing or imminent CRC development.

The afore-described methods have in common that they determine the occurrence of at least one polypeptide of SEQ ID Nos. 1 to 32 either in test samples obtained from human or animal individuals being suspected of suffering from CRC development or directly in human or animal individuals being suspected of suffering from CRC development.

Further, the data obtained for these molecules are then compared with the data obtained either from control samples or control individuals. The comparison with the control samples or control individuals serves to identify these test samples obtained from human individuals being suspected of suffering from CRC or to identify these human individuals being suspected of suffering from CRC in which at least one polypeptide of SEQ ID Nos. 1 to 32 is over-expressed compared to the respective control sample or control individual.

The term "control sample" or "control individual" therefore refers either to a sample obtained from e.g. a human individual or to e.g. a human individual not suffering from CRC cancer. Such individuals may be identified by performing classical CRC diagnosis. A person skilled in the art will be aware that for the purposes of the comparison between a test sample versus control sample and a test individual versus control individual, a proper standardization of the obtained expression data must be undertaken. However, such standardization is common in the art and usually includes determination of expression of polypeptides of SEQ ID Nos. 1 to 32 in relation to e.g. a polypeptide not being involved in colorectal cancer. Thus, in one aspect, data obtained from test samples and control samples may be standardized with respect to expression of a compound such as e.g. actine.

In preferred embodiments of the above mentioned methods of diagnosis or data acquisition, one monitors expression of at least the ten polypeptides of SEQ ID No.1 to 10 and SEQ ID No.: 32, of the polypeptides of SEQ ID No. 1, 2, 3 and 4, or of the polypeptides of SEQ ID No. 1, 2 and 3.

In the following, the present invention will be described with respect to some specific examples. These examples are however not to be construed as being limiting.

### EXAMPLES

### Experiment 1: Identification of cell surface proteins potentially involved in colorectal cancer

Colorectal cancer (CRC) cell lines COLO 205, HT-29, Caco-2, RKO and HCT116 were obtained from the American Type Culture Collection (ATCC). These are all cell lines used as model system for CRC (Lengauer. et al (1997) PNAS 94(6):2545-2550).

All cells except CACO2 were grown in complete medium (Dulbecco's Modified Eagle's Medium, DMEM; Lonza Biowhittaker, Verviers, Belgium) containing 10% Fetal Calf Serum (FCS) and 1% Penicillin/Streptomycin (penicillin 50 units/ml, Astellas Pharma B.V., Leiderdorp, Netherlands; streptomycin 50ug/ml, Fisiopharma, Palomonta (SA), Italy) at 37°C with a CO₂ atmosphere concentration of 5%. The CACO2 cell line was grown in RPMI1640 (Lonza Biowhittaker, Verviers, Belgium) containing 20% of FCS and 1% Penicillin/Streptomycin (penicillin 50 units/ml, Astellas Pharma B.V., Leiderdorp, Netherlands); streptomycin 50ug/ml (Fisiopharma, Palomonta (SA), Italy) at 37°C with a CO₂ atmosphere concentration of 5%.

The cell lines were grown until a confluency of 70/80% was obtained.

Subsequently, the cells were labelled with biotin by incubating the cells at 4°C for 30 min with 412µM Sulfo-NHS-SS-Biotin (Pierce, Rockford, USA) dissolved in PBS.

Following this incubation the cells were washed with PBS and lysed in lysis buffer (Pierce) supplemented with protease inhibitor cocktail (pic) (Complete Protease Inhibitor Cocktail, 1x, Roche, Mannheim, Germany)

The cell lysate was incubated at 4°C for 120 min with NeutrAvidin Protein beads (Pierce) in a rotator to achieve binding of the biotin labelled proteins to the beads. The beads were than washed in two wash buffers. First three times in wash buffer A containing 1% w/v nonidet P-40 and 0.1 % w/v SDS in PBS followed by three times in wash buffer B containing 0.1% w/v nonidet P-40 and 0.5 M NaCl in PBS.

The proteins were than eluted from the beads using PBS containing 50 mM DTT and 62.5 mM Tris HCl. This fraction was designated as cell surface polypeptide fraction.

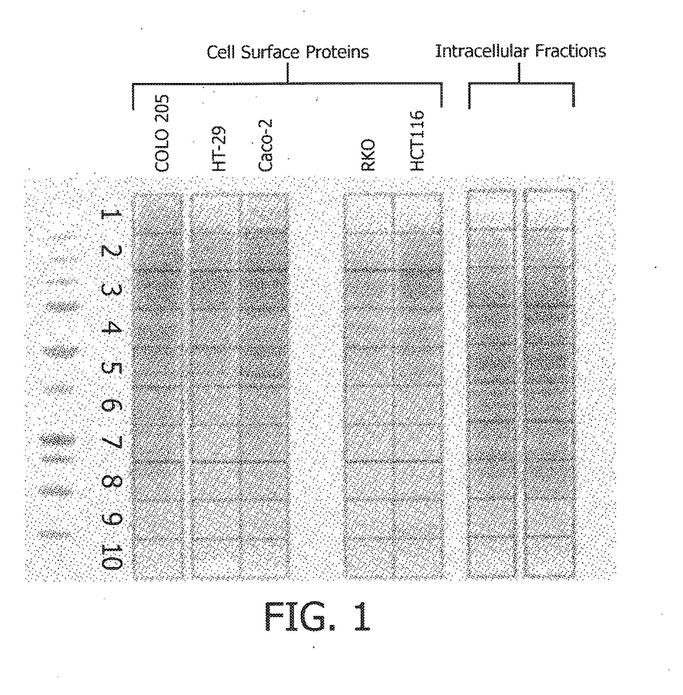

Subsequently, the purified fractions were analysed by SDS-PAGE. An example of biotin-labelled cell surface and intracellular fractions is shown in Fig. 1.

The streptavidine-purified biotin-labelled proteins of the cell surface fraction were then identified by mass spectrometry.

After electrophoresis the gels were fixed in 50% ethanol containing 3% phosphoric acid and stained with Coomassie R-250. After staining the gels were washed in MilliQ water and stored at 4 °C until processing for in-gel digestion.

The gel lanes corresponding to the cell surface proteins of the five different cell lines and the mixtures of intracellular fractions were cut in 10 bands. Each band was processed for in-gel digestion. Briefly, bands were washed dehydrated three times in 50 mM ABC (ammonium bicarbonate pH 7.9)/50 mM ABC + 50% ACN (acetonitrile).

Subsequently, cysteine bonds were reduced with 10mM dithiotreitol for 1h at 56°C and alkylated with 50 mM iodoacetamide for 45 min at RT in the dark. After two subsequent wash/dehydration cycles the bands were dried 10 min in a vacuum centrifuge and incubated overnight with 0.06 µg/µl trypsin at 25 °C. Peptides were extracted once in 1% formic acid and

Subsequently two times in 50% ACN in 5% formic acid. The volume was reduced to 50 µl in a vacuum centrifuge prior to LC-MS/MS analysis.

Peptides were separated by an Ultimate 3000 nanoLC system (Dionex LC-Packings, Amsterdam, The Netherlands) equipped with a 20 cmx75 µm ID fused silica column custom packed with 3 µm 100 Å ReproSil Pur C18 aqua (Dr Maisch GMBH, Ammerbuch-Entringen, Germany). After injection, peptides were trapped at 30 µl/min on a 5 mm×300 µm ID Pepmap C18 cartridge (Dionex LC-Packings, Amsterdam, The Netherlands) at 2% buffer B (buffer A: 0.05% formic acid in MQ; buffer B: 80% ACN+0.05% formic acid in MQ) and separated at 300 nl/min in a 10-40% buffer B gradient in 60 min.

Eluting peptides were ionized at 1.7 kV in a Nanomate Triversa Chip-based nanospray source using a Triversa LC coupler (Advion, Ithaca, NJ). Intact peptide mass spectra and fragmentation spectra were acquired on a LTQ-FT hybrid mass spectrometer (Thermo Fisher, Bremen, Germany). Intact masses were measured at resolution 50.000 in the ICR cell using a target value of 1×10⁶ charges. In parallel, following an FT pre-scan, the top 5 peptide signals (charge-states 2+ and higher) were submitted to MS/MS in the linear ion trap (3 amu isolation width, 30 ms activation, 35% normalized activation energy, Q value of 0.25 and a threshold of 5000 counts). Dynamic exclusion was applied with a repeat count of 1 and an exclusion time of 30 s.

MS/MS spectra were searched against the human IPI database 3.31 (67511 entries) using Sequest (version 27, rev 12), which is part of the BioWorks 3.3 data analysis package (Thermo Fisher, San Jose, CA). MS/MS spectra were searched with a maximum allowed deviation of 10 ppm for the precursor mass and 1 amu for fragment masses. Methionine oxidation and cysteine carboxamidomethylation were allowed as variable modifications. Two missed cleavages were allowed and the minimum number of tryptic termini was 1. After database searching the DTA and OUT files were imported into Scaffold 1.07 (Proteome software, Portland, OR). Scaffold was used to organize the gelband data and to validate peptide identifications using the Peptide Prophet algorithm [14], only identifications with a probabilityN95% were retained. Subsequently, the Protein

Prophet algorithm [15] was applied and protein identifications with a probability of N99% with 2 peptides or more in at least one of the samples were retained. Proteins that contained similar peptides and could not be differentiated based on MS/MS analysis alone were grouped. For each protein identified, the number of unique peptides was exported to Excel. (Piersma et al. (2009) J. Proteomics 72(1): 91-109)

This analysis revealed approximately 2986 proteins.

In parallel, an analysis of genes being implicated in the transgression of adenoma to colorectal adenocarcinoma was performed. This analysis revealed approximately 2478 genes the expression of which is either up- or down-regulated. This approach is described in detail in Carvalho et al. in Gut (2009), 58(1):79-89.

Based on this analysis, only those proteins of the purified cell surface fraction for which the gene analysis data revealed an over-expression were selected. This led to identification of approximately 1305 proteins. Of these 355 genes were over expressed with a significant p-value (>0.05) according to the Wilcox rank test (see Carvalho et al Gut (2009), 58(1):79-89).

In a next step, proteins were selected which comprised at least one transmembrane (TM) domain.

To this end, the software program Phobias developed by the Stockholm Bioinformatics Center was used. (Käll et al. (2004) Journal of Molecular Biology, 338(5):1027-1036). This program indicates whether a protein contains a transmembrane region and/or a signal peptide. Proteins containing either one of these or both were selected.

To reduce the number of false positives we determined which proteins were enriched in the cell surface fractions when compared to the non-biotinylated fractions. A log2 ratio measured from spectral counts (RSC) analysis was carried out. This analysis relied on a label-free quantification approach based on spectral counts. Such an approach is well suited to calculate relative protein abundance between samples and was done according to the method of Old et al. (Old et al (2005) Molecular & Cellular Proteomics 4(10): 1487-1502).

Using this analysis, only proteins with a positive RSC value were selected.

Finally, proteins were selected which were found in at least 70% of the CRC cell lines tested.

This revealed a set of 31 proteins having SEQ ID Nos.: 1 to 32.

A further set of 11 proteins was identified for which the RSC value increased at least by a factor of 2.5. These proteins have SEQ ID Nos. 1 to 10 and SEQ ID No.: 32.

Of these, histological analysis was performed for PRNP (SEQ ID No.: 6) and BCAM (SEQ ID No.: 5) on tumor material obtained from CRC patients.

A 4 mm section of formalin fixed paraffin embedded (FFPE) tissue was used for immunohistochemistry. After deparaffination in xylene, and rehydration through graded alcohol to water, endogenous peroxidase was blocked with hydrogen peroxide (0.3% H2O2/methanol) for 30 min. Antigen retrieval was done by autoclaving in 1.5mM HCL for PRNP and TRIS/EDTA ph 9 buffer for BCAM. Primary anti-prion mouse clone 8H4 antibody from sigma-aldrich (St. Louis,MO,USA) was incubated overnight at a dilution of 1:100 and mouse anti human CD239 from abD serotech (Oxford,UK) was also incubated overnight at a dilution of 1:100. Following extensive washing the slides were incubated for 30 min with poly-HRP-goat anti-mouse/rabbit/rat IgG (Powervision, Immunologic, Duiven). Then all sections were washed and incubated with DAB Substrate-Chromogen (Dako, Glostrup, Denmark). Counterstaining was done with Mayer's haematoxylin. Incubation without primary antibody was used as negative control.

The results can be seen in Fig. 2 for PRNP and Fig. 3 for BCAM.

## Claims

1. A contrast agent for use in MRI and/or MPI for diagnosing ongoing or imminent CRC development, said contrast agent comprising at least one compound being capable of interacting with a polypeptide of SEQ ID No.: 1, said at least one compound being selected from antibodies which specifically interact with the polypeptide of SEQ ID No.: 1, wherein said at least one compound is coupled to a detectable marker molecule, said detectable marker molecule being selected from the group consisting of USPIOS and 19Fluor.

2. A contrast agent according to claim 1 for use according to claim 1, wherein the antibody is selected from commercially available antibodies which specifically interact with the said polypeptide.

3. A contrast agent according to claim 1 for use according to claim 1 for use differentiating progressive (high-risk) CRC (adenocarcinomas) from non-progressive (low-risk) colorectal adenomas.

## Patentansprüche

1. Kontrastmittel zur Verwendung bei der Magnetresonanzbildgebung (MRI) und/oder Magnetpartikelbildgebung (MPI) zur Diagnose der weiteren oder bevorstehenden Entwicklung eines kolorektalen Karzinoms, wobei das genannte Kontrastmittel mindestens eine Verbindung umfasst, die in der Lage ist, mit einem Polypeptid der SEQ. ID Nr. 1 zu interagieren, wobei die genannte mindestens eine Verbindung aus Antikörpern ausgewählt wird, die speziell mit dem Polypeptid der SEQ. ID Nr. 1 interagieren, wobei die mindestens eine Verbindung mit einem nachweisbaren Markermolekül gekoppelt ist, wobei das genannte nachweisbare Markermolekül aus der Gruppe bestehend aus USPIOS und 19Fluor ausgewählt wird.

2. Kontrastmittel nach Anspruch 1 zur Verwendung nach Anspruch 1, wobei der genannte Antikörper aus handelsüblichen Antikörpern ausgewählt wird , die speziell mit dem genannten Polypeptid interagieren.

3. Kontrastmittel nach Anspruch 1 zur Verwendung nach Anspruch 1 zum Unterscheiden progressiver (mit hohem Risiko verbundener) kolorektaler Karzinome (Adenokarzinome) von nicht-progressiven (mit geringem Risiko verbundener) kolorektalen Adenomen.

## Revendications

1. Agent de contraste destiné à être utilisé dans une IRM et/ou une IPM pour diagnostiquer un développement de CRC en cours ou imminent, ledit agent de contraste comprenant au moins un composé capable d'interagir avec un polypeptide de SEQ ID No.: 1, ledit au moins un composé étant sélectionné parmi des anticorps qui interagissent spécifiquement avec le polypeptide de SEQ ID No.: 1, dans lequel ledit au moins un composé est couplé à une molécule de marqueur détectable, ladite molécule de marqueur détectable étant sélectionnée dans le groupe se composant de USPIOS et 19Fluor.

2. Agent de contraste selon la revendication 1 destiné à être utilisé selon la revendication 1, dans lequel l'anticorps est sélectionné parmi des anticorps disponibles dans le commerce qui interagissent spécifiquement avec ledit polypeptide.

3. Agent de contraste selon la revendication 1 destiné à être utilisé selon la revendication 1 pour différencier un CRC (adénocarcinomes) progressif (à haut risque) d'adénomes colorectaux non progressifs (à bas risque).
